# EUROPÄISCHE PATENTSCHRIFT

(11) **EP 1 483 588 B1**
(45) Veröffentlichungstag und Bekanntmachung des Hinweises auf die Patenterteilung: **10.03.2010**
(21) Anmeldenummer: 03706616.4
(22) Anmeldetag: 14.03.2003
(51) Int. Cl.: G01N 33/72, C12Q 1/68, C12N 15/00, C12N 5/00, A61K 38/42, A61K 48/00

(54) **SCREENINGVERFAHREN ZUM IDENTIFIZIEREN PROTEKTIVER SUBSTANZEN FÜR DIE BEHANDLUNG NEURODEGENERATIVER UND/ODER ISCHÄMISCHER ERKRANKUNGEN**
SCREENING METHOD FOR IDENTIFYING PROTECTIVE SUBSTANCES FOR TREATING NEURODEGENERATIVE AND/OR ISCHEMIC DISORDERS
PROCEDE DE TRI PERMETTANT L'IDENTIFICATION DE SUBSTANCES PROTECTRICES EN VUE DU TRAITEMENT DE MALADIES NEURODEGENERATIVES ET/OU ISCHEMIQUES

(30) Priorität: 14.03.2002 DE 10211369
(43) Veröffentlichungstag der Anmeldung: 08.12.2004
(73) Patentinhaber: Sygnis Bioscience GmbH & Co. KG, 69120 Heidelberg (DE)
(72) Erfinder: MAURER, Martin, 69121 Heidelberg (DE); KUSCHINSKY, Wolfgang, 69118 Heidelberg (DE); SCHNEIDER, Armin, 69118 Heidelberg (DE); BACH, Alfred, 68535 Edingen-Neckarhausen (DE)
(74) Vertreter: Isenbruck, Günter
(86) Internationale Anmeldenummer: PCT/EP2003/002718
(87) Internationale Veröffentlichungsnummer: WO 2003/076950

(56) Entgegenhaltungen:
- DOVER G J ET AL: "HYDROXYUREA INDUCTION OF HEMOGLOBIN F PRODUCTION IN SICKLE CELL DISEASE RELATIONSHIP BETWEEN CYTOTOXICITY AND F CELL PRODUCTION" BLOOD, Bd. 67, Nr. 3, 1986, Seiten 735-738, XP009010479 ISSN: 0006-4971
- BRINES MICHAEL L ET AL: "Erythropoietin crosses the blood-brain barrier to protect against experimental brain injury." PROCEEDINGS OF THE NATIONAL ACADEMY OF SCIENCES OF THE UNITED STATES, Bd. 97, Nr. 19, 12. September 2000 (2000-09-12), Seiten 10526-10531, XP002240921 September 12, 2000 ISSN: 0027-8424
- BROYLES R H ET AL: "Specific repression of beta-globin promoter activity by nuclear ferritin" PROCEEDINGS OF THE NATIONAL ACADEMY OF SCIENCES OF USA, NATIONAL ACADEMY OF SCIENCE. WASHINGTON, US, Bd. 98, Nr. 16, 31. Juli 2001 (2001-07-31), Seiten 9145-9150, XP002218772 ISSN: 0027-8424
- D'ARCANGELO G. ET AL: 'ACTIVATION OF CODEPENDENT TRANSCRIPTION FACTORS IS REQUIRED FOR TRANSCRIPTIONAL INDUCTION OF THE VGF GENE BY NERVE GROWTH FACTOR AND RAS', 01 September 1996, MOLECULAR AND CELLULAR BIOLOGY, AMERICAN SOCIETY FOR MICROBIOLOGY, WASHINGTON, US, PAGE(S) 4621 - 4631, ISSN 0270-7306 XP009010403
- RUSCHITZKA F.T. ET AL: 'NITRIC OXIDE PREVENTS CARDIOVASCULAR DISEASE AND DETERMINES SURVIVAL IN POLYGLOBULIC MICE OVEREXPRESSING ERYTHROPOIETIN', 10 Oktober 2000, PROCEEDINGS OF THE NATIONAL ACADEMY OF SCIENCES OF USA, NATIONAL ACADEMY OF SCIENCE, WASHINGTON, DC., US, PAGE(S) 11609 - 11613, ISSN 0027-8424 XP001148012

## Beschreibung

Die vorliegende Erfindung betrifft ein Screeningverfahren zum identifizieren protektiver Substanzen, die die Hämoglobinbildung in neuronalen Zellen beeinflussen, sowie rekombinante Konstrukte, Wirtszellen und transgene Tiere zur Durchführung dieses Verfahrens. Ferner betrifft die vorliegende Erfindung ein Diagnoseverfahren zur Differentialdiagnose neurodegenerativer und/oder ischämischer Erkrankungen in Säugetieren und die Verwendung von Hämoglobin, einem Globin oder einem Mutein oder Fusionsprotein davon oder eine entsprechende Nukleinsäure zur Behandlung neurodegenerativer und/oder ischämischer Erkrankungen in Säugetieren. Die Erfindung betrifft weiterhin Konstrukte zur Gentherapie bei solchen Erkrankungen.

Nach der Statistik des Statistischen Bundesamtes verursachen Himgefäßerkrankungen jedes Jahr etwa 90.000 Todesfälle in der Bundesrepublik Deutschland. Die Zahlen zu Beginn der 1980er Jahre weisen aus, dass etwa die Hälfte der Schlaganfallpatienten die ersten drei Wochen nicht überteben. Obwohl die Zahl der Neuerkrankungen z.Zt. sinkt, bleibt der Schlaganfall neben dem Herzinfarkt eine der bedeutendsten Erkrankungen in den mittleren Altersgruppen.

Bei dem Schlaganfall handelt es sich um eine nach flüchtigen Vorläufern schlagartig einsetzende schwere Funktionsstörung des Gehirns durch Verminderung oder vollständige Unterbrechung der Blutversorgung umschriebener Gehirnbezirke, verbunden mit Bewußtlosigkeit, Lähmungserscheinungen sowie Sprach- und Gefühlsstörungen. Dank der guten medizinischen Versorgung überleben vom Schlaganfall Betroffene heute länger als früher, jedoch müssen von diesen ca. 50-60% mit einer Behinderung aufgrund neurologischer Schäden leben.

Erythropoietin (EPO) ist ein saures Glycoprotein, das ursprünglich als hämatopoetischer Faktor im Serum gebluteter Kaninchen gefunden wurde. Es bewirkt die Induktion der Erythropoese, um Gewebehypoxien entgegenzuwirken. In dieser Funktion wurde es schon länger zur Therapie von Anämien herangezogen, z.B. bei Dialysepatienten. Von dieser erythropoetischen Wirkung abgesehen, wurde in jüngerer Zeit die Aufmerksamkeit auf Wirkungen von EPO im zentralen Nervensystem gelenkt. Eine funktionelle Wirkung von EPO auf das Gehirn, insbesondere eine neuroprotektive Wirkung, wurde in jüngster Zeit eindeutig belegt (Brines, et al., 2000. Proc Natl Acad Sci USA 97, 10526-31; Digicaylioglu und Lipton, 2001, Nature 412, 641-7). Dover (Blood, Bd. 67, Nr. 3, 1986, Seiten 735-738) offenbart die Identifikation von 5-Azacytidin und Hydroxyurea als Substanzen, welche die Fetal-Hämoglobin-Produktion erhöhen und so die Schwere einer Sichelzellanämie vermindern Reticulozyten sind die Testzellen. Das Dokument offenbart jedoch nicht neuronale Zellen als Testzellen. Brines (PNAS, Bd 97, Nr. 19, 2000, Seiten 10526-10531) beschreibt die neuroprotektive Funktion von EPO während hypoxischem oder ischemischem Stress. EPO erhöht die Masse der roten Blutkörperchen und wird für neuroprotektive Behandlungen eingesetzt. Neuronale Zellen werden nicht offenbart. Dies schließt protektive Wirkungen in Modellen neurologischer Erkrankungen wie cerebrale Ischämien, experimentell induzierte Enzephalomyelitis oder Subarachnoidalblutungen ein. Die Bedeutung von Erythropoietin für das Überleben von Neuronen unter hypoxisch-ischämischen Zuständen ist in zahlreichen Studien in vivo (Bemaudin et al., 1999, J. Cereb. Blood Flow Metab. 19, 643-651; Brines et al., 2000, a.a.O.) und in vitro (Siren et al., 2001, Proc. Natl. Acad. Sci. U.S.A. 98, 4044-4049; Digicaylioglu und Lipton, 2001, Nature 412, 641-647) belegt.

Wegen seiner erythropoetischen Wirkung bewirkt die Gabe von EPO jedoch neben der neuroprotektiven Wirkung eine Erhöhung des Hämatokrits, die zum Teil der neuroprotektiven Wirkung entgegensteht, denn durch die Erhöhung des Hämatokrits verschlechtern sich die rheologischen Eigenschaften des Blutes, was sich negativ auf die Mikrozirkulation auswirkt. Es besteht also ein Bedarf an Substanzen mit der protektiven Wirkung von EPO, ohne jedoch seine unerwünschten Nebenwirkungen.

Es ist deshalb eine Aufgabe der vorliegenden Erfindung, ein Screeningverfahren bereitzustellen, das die Identifizierung neuer Substanzen mit protektiver Wirkung auf neuronale, myokardiale und/oder Skelettmuskelzellen erlaubt.

Diese Aufgabe wird gelöst von einem Screeningverfahren zum Identifizieren protektiver Substanzen, die die Hämoglobinbildung in neuronalen, myokardialen und/oder Skelettmuskelzellen Zellen beeinflussen, umfassend das Inkontaktbringen der zu testenden Substanz mit einer neuronalen Testzelle, und das Nachweisen einer veränderten Hämoglobinbildung in der Testzelle Broyles (PNAS, Bd. 98, Nr. 16, 2001, Seiten 9145-9150) bespricht Chloramphenicolacetyltransferase-Reporter-Plasmide, die einen beta-Globin-Reporter beinhalten, werden in epitheliale (CV-1-) Zellen transfiziert. Neuronale Zellen werden nicht offenbart. D'Arcangelo (Mol Cell Biol, Bd. 16, Nr. 9, 1996, Seiten 4621-4631) offenbart PC12-Zellen (neuronale Zellen), die mit einem beta-Globin-Reportergen transfiziert wurden (Zusammenfassung). Hämoglobinbildung in neuronalen Zellen ist nicht besprochen.

Das erfindungsgemäße Screeningverfahren beruht auf einem in Säugetieren bisher unbekannten protektivem Prinzip, das mit einer differentiellen Regulation von alpha- und beta-Globin im zentralen Nervensystem in Zusammenhang steht. Es wurde überraschenderweise festgestellt, dass im Gehirn von transgenen Mäusen, die humanes Erythropoietin überexprimieren (Linien tg6 und tg21; Ruschitzka et al., 2000, Proc Natl Acad Sci USA 97, 11609-13), eine verstärkte Expression von Hämoglobin sowohl auf der Boten-RNA-Ebene als auch auf der Protein-Ebene stattfindet. Die in den Beispielen beschriebenen Ergebnisse zeigen, dass im Gehirn der transgenen Tiere Boten-RNA für Hämoglobin etwa um den Faktor 3-4 induziert ist und Hämoglobin-Protein um den Faktor 2-3. Eine Regulation und Induktion von Hämoglobin in Hirnzellen war bisher weder auf RNA- noch auf Protein-Ebene beschrieben worden Ruschitzka (PNAS, Bd. 97, Nr. 21, 2000, Seiten 11609-11613) offenbart transgene Mäuse, in welchen humane Epo cDNA mit einem Promoter, welcher vor allem zu Transgenexpression in neuronale Zellen führt, überexprimiert ist. Der erhöhte plasmatische Epo-Level im Gehirn führt zu doppelter Menge an Hämoglobin im Vergleich zu Mäusen ohne transgene Veränderung (S 11610, Spalte 1-2, übergreifender Abs). Es ist kein Screeningverfahren zum Identifizieren von für neuronale Zellen protektive Substanzen, die die Hämoglobinbildung beeinflussen, offenbart. Explizit wird Hämoglobinbildung innerhalb von neuronalen Zellen auch nicht besprochen.

Der Begriff "protektive Substanzen" bezeichnet Substanzen, die in vitro (in Zellkultursystemen) und/oder in vivo (in Tiermodellen ischämischer, hypoxischer und/oder neurodegenerativer Erkrankungen, wie z.B. Schlaganfallmodellen, Modellen für amyotrophe Lateralsklerose oder Modellen für die Parkinson'sche Erkrankung) und/oder beim Menschen protektive Wirkung auf Zellen und/oder auf einzelne Organe als Ganzes (z.B. Gehirn oder Herz) haben. Dabei kann die Applikation der Substanz vor oder nach dem schädigenden Ereignis stattfinden.

Ein Test auf protektive Wirkung kann z.B. ein in vitro-Test mit kultivierten Testzellen, die einer schädigenden Noxe ausgesetzt werden, z.B. einer hypoxischen Behandlung, einer Behandlung mit H₂O₂, Glutamat, MPP oder anderen schädigenden Substanzen. Ein üblicher in vitro-Test für protektive Substanzen ist die Vabilitätsuntersuchung von Zellen, in denen das Überleben bzw. die Schädigung der Zelle untersucht wird, wie in Lindl, 2000, Zell- und Gewebekultur, Spektrum Akad. Vlg., Heidelberg; und Sinor und Greenberg, 2000, Neurosci. Lett. 290, 213-215 beschrieben; andere Tests schließen Untersuchungen auf Expressionapoptotischer Marker ein (Siren et al., 2001, Proc. Natl. Acad. Sci. USA 98, 4044-4049) oder bestimmen eine Verminderung des Infarktvolumen als Mass für eine neuroprotektive Wirkung (Brines et al., 2000, a.a.O.).

Der Test kann auch in vivo, z.B. in Mäusen erfolgen, die mit der zu testenden Substanz behandelt werden und danach einer Schädigung ausgesetzt werden, beispielsweise in einem Schlaganfall- oder Herzinfarktmodell. In einem Schlaganfallmodell kann die schützende Wirkung z.B. anhand einer Verringerung des Infarktvolumens festgestellt werden. Entsprechend auch für das Herzinfarktmodell (siehe Brines et al, 2000, a.a.O.)

Unter "neuronaler (Test)zelle" werden Zellen verstanden, die neuronalen Ursprungs sind oder neuronenähnliches Wachstums- und Differenzierungsverhalten aufweisen, d. h. wesentliche Merkmale von natürlich vorkommenden Nervenzellen besitzen, oder von Zellen abgeleitet sind, die neuronale Identität haben, wie z. B. PC12-Zellen oder Neuroblastom-Zellen.

Der Begriff "veränderte Hämoglobinbildung" bezeichnet eine Erhöhung oder Senkung der Menge an Hämoglobin auf Globin-RNA- und Hämoglobin-Protein-Ebene in der Testzelle, wobei der Unterschied bezogen auf unbehandelte Kontrollzellen ein Signifikanzniveau von 5 % aufweisen muss.

Die mit der protektiven Wirkung in Zusammenhang stehende Regulation von Hämoglobin in neuronalen Zellen kann für therapeutische Zwecke ausgenutzt werden, um Substanzen zu finden, die über eine Hoch- bzw. Niederregulation des Hämoglobins zu einem Schutz von neuronalen Zellen unter schädigenden Einflüssen, z.B. Ischämie, führen. Der prinzipielle Aufbau eines solchen Screeningsystems zur Identifizierung protektiver Substanzen, die zu einer veränderten Hämoglobinbildung in neuronalen Zellen führen, besteht aus 1.) Zellen, die auf Stimuli mit Hämoglobininduktion reagieren; 2.) einer Zugabeeinheit für verschiedene zu testende Substanzen aus einer Substanzbank; und 3.) einem Nachweisverfahren für Globin-RNA oder Hämoglobin-Protein.

Die erfindungsgemäßen Screeningsysteme erlauben das Durchsuchen von chemischen Bibliotheken nach Substanzen, die hemmende oder aktivierende Wirkungen auf die Hämoglobinbildung haben. Die Identifizierung solcher Substanzen stellt den ersten Schritt auf dem Weg zur Identifizierung neuartiger Medikamente dar, die spezifisch auf die Hämoglobin-assoziierte Protektion wirken.

Ein Nachweis von Hämoglobin ist auf verschiedene Weise denkbar. Prinzipiell kann der Nachweis auf RNA- oder Proteinebene erfolgen. In einer Ausführungsform der Erfindung erfolgt der Nachweis der veränderten Hämoglobinbildung durch Nachweis mindestens einer Globin-RNA, vorzugsweise alpha-Globin- und/oder beta-Globin-RNA. Im Sinne der Erfindung steht der Begriff "Globin" für ein Protein der Globin-Familie, die unter anderem das alpha-Globin, das beta-Globin und die fötalen Hämoglobin-Untereinheiten epsilon, zeta, eta und delta (Grosveld et al., 1993, Baillieres Clin Haematol 6, 31-55) umfasst. Eine Übersicht über die Mitglieder der Globin-Familie geben Bumester et al. in Nature 407. 520-23 (2000).

Der RNA-Nachweis kann dabei prinzipiell über eine Reverse Transkription / Polymerasekettenreaktion erfolgen, z.B. mit Hilfe eines PCR-Cyders mit Quantifizierungsmöglichkeit unter SYBR-Green Einbau. Dies kann im 96 oder 384 well-Masstab erfolgen, nach Zugabe der zu testenden Substanz zu neuronalen Zellen wie z.B. PC12-Zellen, abgeleiteten Neuroblastomzellen oder auch primären Neuronen. Dabei kann die relative Quantifizierung der Globin-RNA gegenüber Standardgenen wie z.B. Cyclophilin oder S26 erfolgen. Ebenso wäre ein Nachweis über Hybridisierungsverfahren mit einer geeigneten Sonde, z.B. über Dotblot-Hybridisierung möglich.

Ein Proteinnachweis kann z.B. über spezialisierte Proteinmikrochips erfolgen, die sehr geringe Mengen Hämoglobin-Protein detektieren können (z.B. Mikrochip der Fa. Zyomyx, Hayward, CA, USA), auch unter Zuhilfenahme von Mikrokapillarelektrophorese. Für den Proteinnachweis können auch spezielle Detektionseigenschaften des Hämoglobins herangezogen werden, wie z.B. die Anfärbbarkeit mit Hilfe von 2,7-Diaminofluoren (DAF) oder mit Hilfe der Cyanhämiglobin-Schnellmethode. Der Nachweis von Hämoglobin könnte in primären Zellysaten mit Hilfe photometrischer Methoden auch im Hochdurchsatz durchgeführt werden.

Eine Alternative zum direkten Nachweis einer veränderten Hämoglobinbildung in neuronalen Zellen kann die Benutzung eines Konstruktes darstellen, das neben einem Reportergen wesentliche Bestandteile der Globinregulation auf Stimuli enthält. In einer alternativen Ausführungsform umfasst das erfindungsgemäße Screeningverfahrens den Schritt des Inkontaktbringens der zu testenden Substanz mit einer Testzelle, ausgewählt aus einer neuronalen Zelle die ein Reportergen unter der funktionellen Kontrolle von regulatorischen Elementen mindestens eines Globin-Gens enthält, und den Schritt des Nachweisens des Reportergen-Produktes in der Testzelle.

Die regulatorischen Elemente können Promotorabschnitte und Enhancerelemente sein, die im 5'- oder 3'-Bereich eines Globin-Gens oder in dessen Introns liegen können. Diese Regulationselemente sind mit mindestens einem Reportergen verknüpft, das eine Messung einer veränderten Proteinbildung erlaubt. Bevorzugt handelt es sich hierbei um einfache Meßmethoden (kolorimetrische, luminometrische, auf Fluoreszenz beruhende oder radioaktive), die die schnelle Messung einer Vielzahl von Testsubstanzen erlauben (siehe z.B. Böhm, Klebe, Kubinyi, 1996, Wirkstoffdesign, Spektrum-Veriag. Heidelberg). Geeignete Reportergene sind beispielsweise Luciferase, alkalische Phosphatase (vorzugsweise als sezemierte alkalische Phosphatase), beta-Galactosidase, EGFP oder ähnliche Reportergene, die dem Fachmann geläufig sind. Vorzugsweise werden diese Konstrukte in Zellen exprimiert, die Neuronen-ähnliche Eigenschaften besitzen, z.B. Neuroblastomzellinien oder PC12 Zellen.

Die nachstehenden Figuren und Beispiele erläutern die Erfindung:

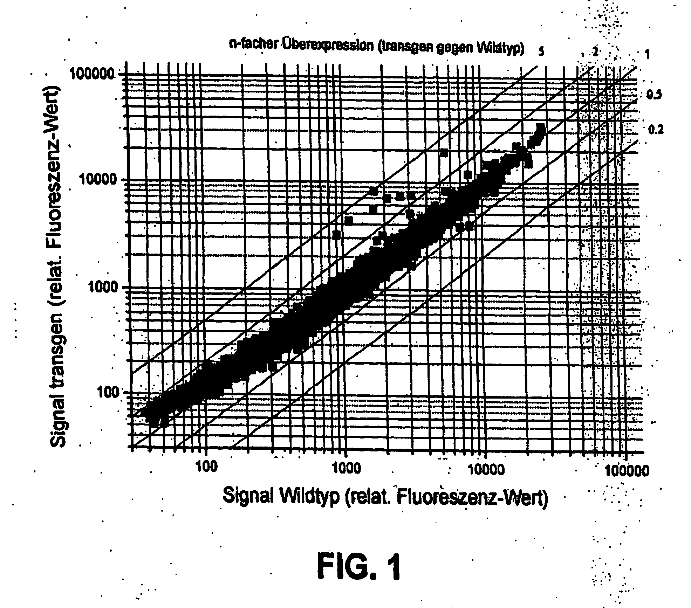

Fig. 1 zeigt in Form eines Diagramms das Ergebnis einer Transkriptionsanalyse im Gehirn von EPO-transgenen Mäusen. Die Grafik stellt die Daten eines DNA-Array Hybridisierungsexperimentes dar. Die Punkte oberhalb der Diagonalen repräsentieren hochregulierte Genprodukte im Gehirn von EPO-transgenen Tieren.

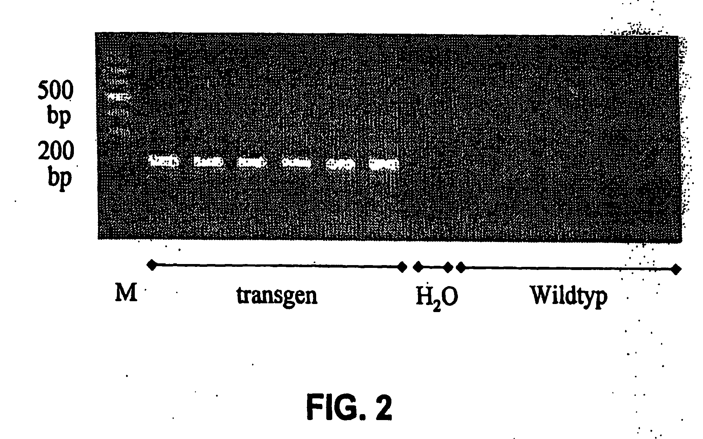

Fig. 2 zeigt das Ergebnis einer Agarose-Gelelektrophorese der RT-PCR-Produkte für Hämoglobin-Boten-RNA Das Bild der Agarose-Gelelektrophorese des RT-PCR-Produkts für alpha-Globin zeigt eine hohe Expression von Hämoglobin-alpha Boten-RNA im Gehirn der sechs transgenen Tieren in Vergleich zu den Wildtyp-Tieren. Diese Analyse ist eine Endpunktanalyse, und deshalb semiquantitativ.

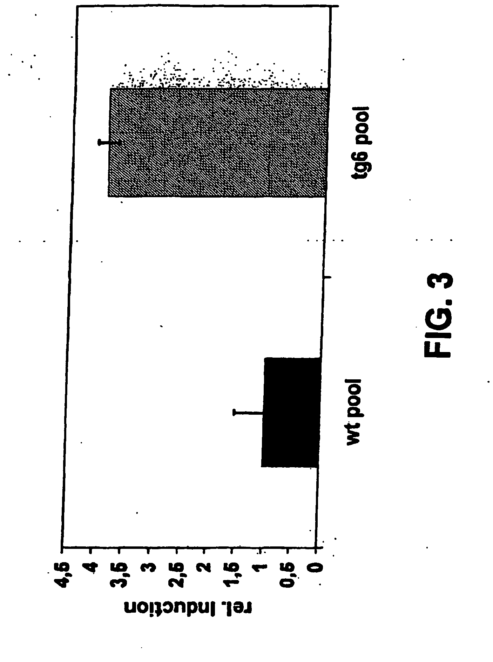

Fig. 3 zeigt das Ergebnis einer quantitativen PCR für alpha-Globin aus Him-cDNA. Die Daten repräsentieren gepoolte RNA aus 6 Hirnen (tg oder wt).

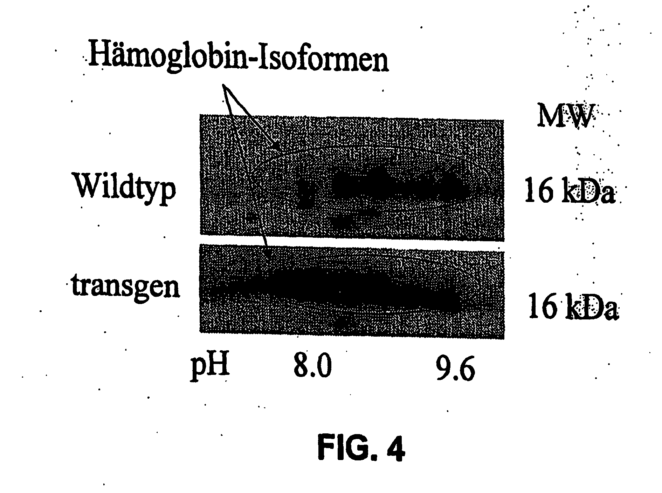

Fig. 4 zeigt das Ergebnis einer zweidimensionalen Gelelektrophorese von Gesamthimextrakt aus Wildtyp- und transgenen Mäusen für Hämoglobin-Proteine. Bei Auftragung der gleichen Menge an Gesamtprotein aus einem Gesamthimextrakt ergeben sich aus dem Vergleich der optischen Dichten und Volumina der Proteinpunkte mittlere Induktionsfaktoren von 2,68 ± 0,29 (n = 6) für Hämoglobin-alpha und 2,59 ± 0,37 (n = 6) für Hämoglobin-beta.

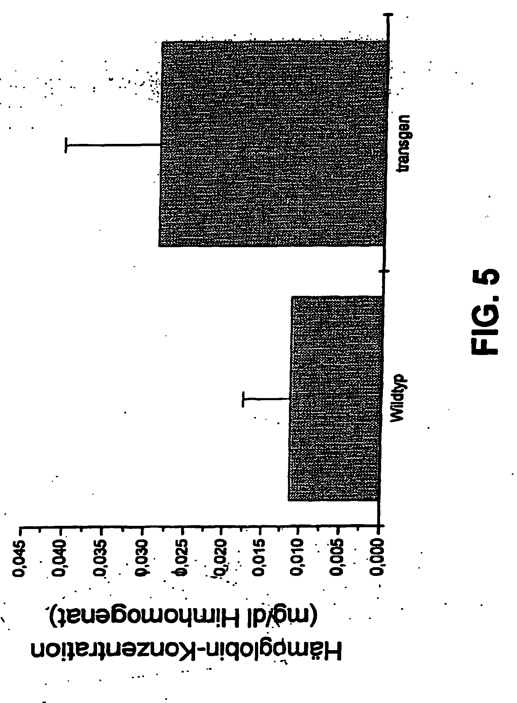

Fig. 5 zeigt ein Balkendiagramm der mittels der Cyanohämiglobin-Methode bestimmten Hämoglobin-Konzentration in dem Himhomogenat von jeweils 6 Wildtyp- und transgenen Mäusen. Die Konzentrationen betrugen 0,029 ± 0,012 mg/(dl Himhomogenat) (n = 6) für die transgenen und 0,011 ± 0,006 mg/(dl Himhomogenat) (n = 6) für die Wildtyp-Gruppe.

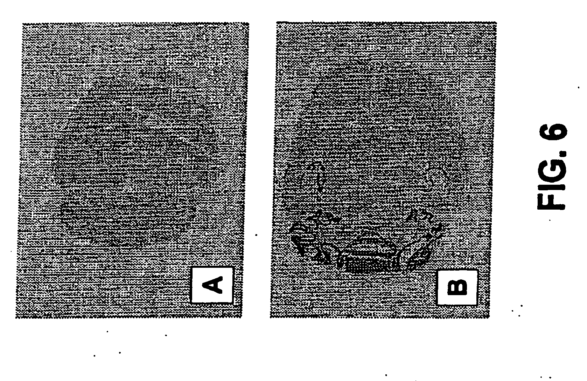

Fig. 6 zeigt das Ergebnis einer in-situ-Hybridisierung zur Lokalisierung von Hämoglobin Boten-RNA im Gehirn. Digoxigenin-markierte Oligonukleotid-Sonden spezifisch für Hämoglobin wurden in Maushim-Kryoschnitten durch Anti-Digoxigenin-Antikörper detektiert und durch eine Farbreaktion sichtbar gemacht (A) zeigt das Gehirn eines nicht-transgenen Geschwistertiers, (B) die deutlich stärkeren in-situ-Signale v.a. in Hippocampus, Gyrus dentatus, Kleinhim und Cortexgebieten.

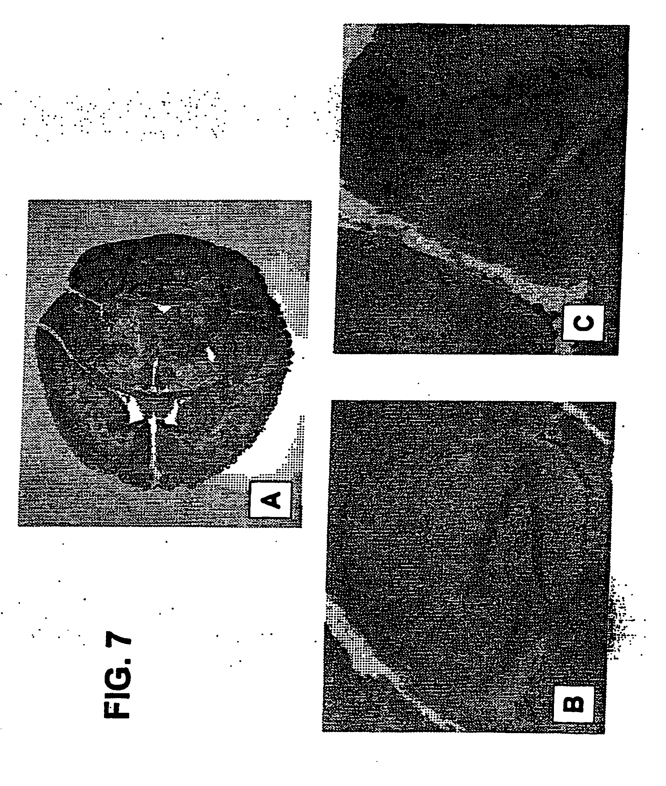

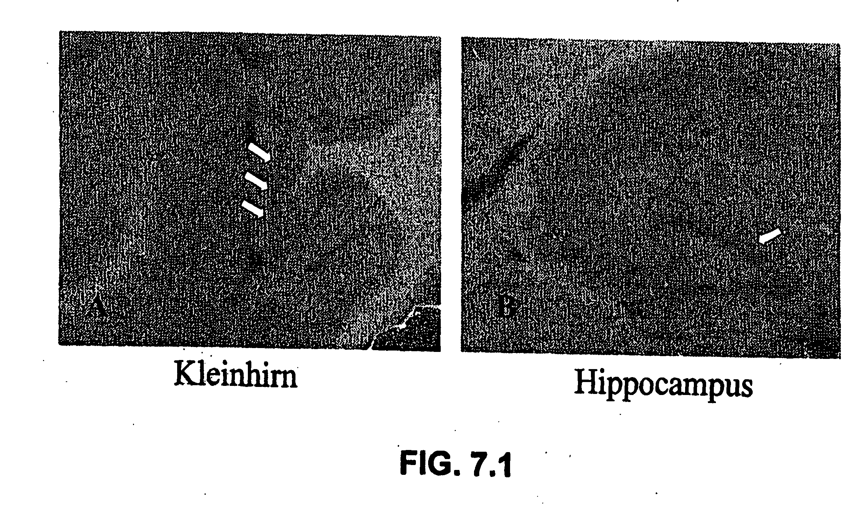

Fig. 7 zeigt die Lokalisierung von Hämoglobin-Protein im Gehirn mit DAF-Färbung. A. Hämoglobinexpression im Gehirn von Mäusen (nicht transgen). Es zeigen sich Signale im Hippocampus, der Kleinhirnrinde und dem paraventrikulären thalamischen Kern. Die Expressionsorte des Hämoglobin-Proteins entsprechen insgesamt den Expressionsorten der Hämoglobin-Boten-RNA. B und C: Vergrösserungen von Kleinhrin und Hippocampus, die die stärkste Expression aufweisen.
Fig. 7.1 Diaminofluorenfärbung für Hämoglobin im Hirnschnitt transgener Mäuse. Es ist eine deutliche Anfärbung (schwarz-blau) im Kleinhirnkortex (A) und Hippocampus (B) zu sehen (Pfeile).

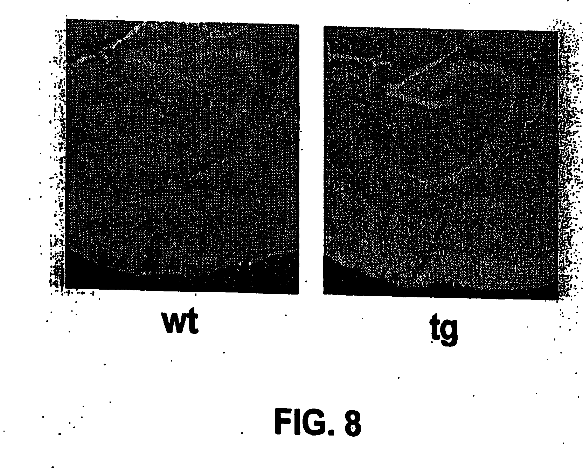

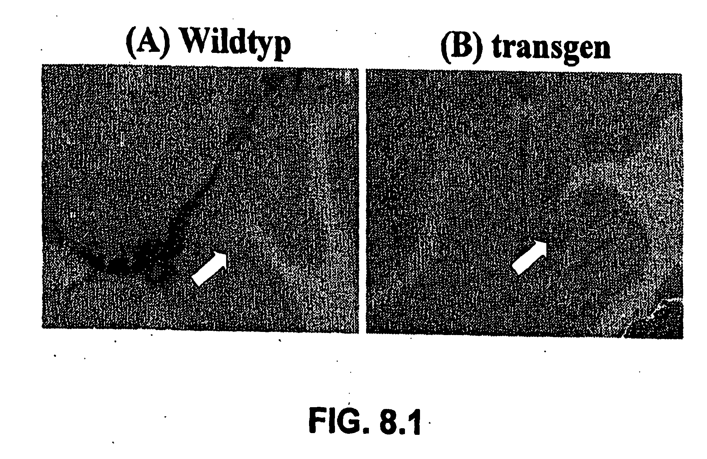

Fig. 8 zeigt die Lokalisierung von Hämoglobin-Protein im Gehirn im Hippocampus von wt- und tg-Tieren mit DAF-Färbung. A. Hämoglobinexpression im Hippocampus von Mäusen (nicht transgen). B. Gleiche Region aus einem transgenen Tier der Linie tg6. Es zeigt sich ein deutlich stärkeres und spezifischeres Signal als in den Wildtyp-Tieren.
Fig. 8.1 Vergleich der Diaminofluorenfärbung für Hämoglobin im Gehirn (A) einer Wildtyp- und (B) einer transgenen Maus. Im Gehirn der transgenen Maus findet sich eine deutlich höhere Expression (schwarz-blau); gezeigt ist ein Abschnitt aus dem Kleinhirnkortex.

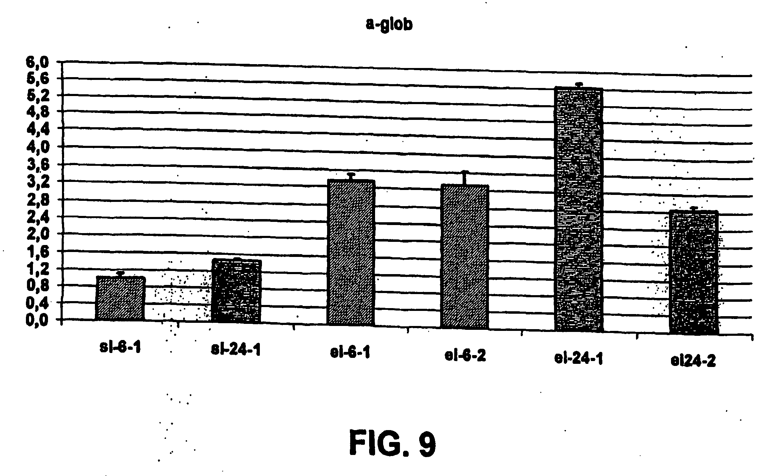

Fig. 9 zeigt in Form eines Balkendiagramms die Ergebnisse einer quantitativen PCR:alpha-Globin-Induktion nach intraperitonealer EPO-Gabe.Gezeigt ist die Hochregulation von alpha-Globin-Boten-RNA 6 und 24 Stunden nach i.p. EPO-Gabe in Hemisphärenhälften von Ratten. si-6-1: sham-injiziertes Tier 6 h nach NaCl-Gabe; si-24-1: sham-injiziertes Tier 24 h nach NaCl-Gabe; ei-6-1 / ei-6-2: EPO-injizierte Tiere 6 h nach EPO-Gabe, ei-24-1 / ei-24-2: EPO-injizierte Tiere 24 h nach EPO-Gabe.

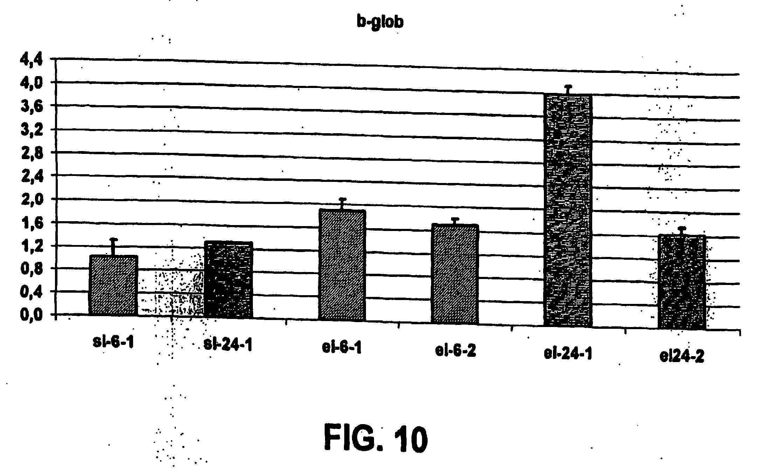

Fig. 10 zeigt in Form eines Balkendiagramms die Ergebnisse einer quantitativen PCR: beta-Globin-Induktion nach intraperitonealer EPO-Gabe. Gezeigt ist die Hochregulation von alpha-Globin-Boten-RNA 6 und 24 Stunden nach i.p. EPO-Gabe in Hernisphärenhälften von Ratten. Si-6-1: sham-injiziertes Tier 6 h nach NaCl-Gabe; si-24-1: sham-injiziertes Tier 24 h nach NaCl-Gabe; ei-6-1 / ei-6-2: EPO-injizierte Tiere 6 h nach EPO-Gabe, ei-24-1 / ei-24-2: EPO-injizierte Tiere 24 h nach EPO-Gabe.

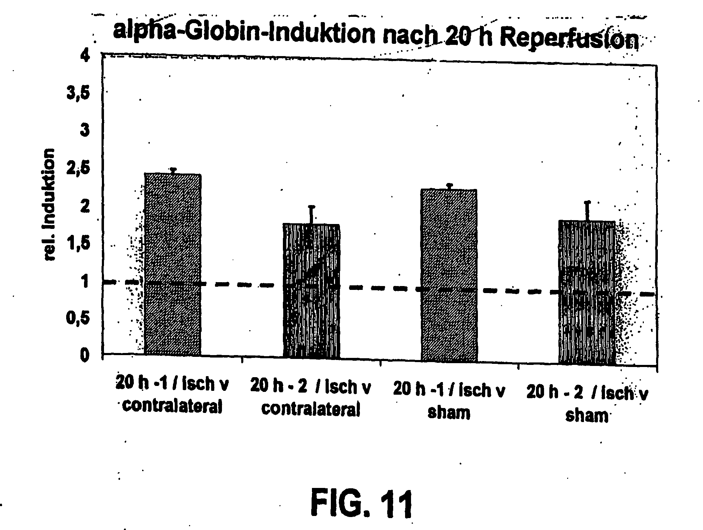

Fig. 11 zeigt in Form eines Balkendiagramms die Ergebnisse eines Versuchs zur Bestätigung der Hochregulation von alpha-Globin mittels quantitativer PCR. Mit alpha-Globin spezifischen Primern wurde die relative Regulation der mRNA gegenüber der kontralateralen Hemisphäre und sham-operierten Tieren bestimmt Die Fehlerbalken geben die Standardabweichung einer Messung über mehrere Verdünnungsstufen an.

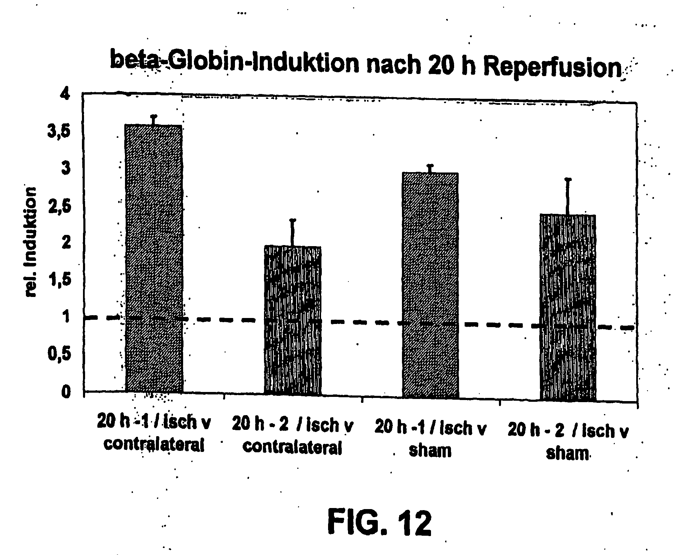

Fig. 12 zeigt in Form eines Balkendiagramms die Ergebnisse eines Versuchs zur Bestätigung der Hochregulation von beta-Globin mittels quantitativer PCR. Mit beta-Globin spezifischen Primem wurde die relative Regulation der mRNA gegenüber der kontralateralen Hemisphäre und sham-operierten Tieren bestimmt Die Fehlerbalken geben die Standardabweichung einer Messung über mehrere Verdünnungsstufen an.

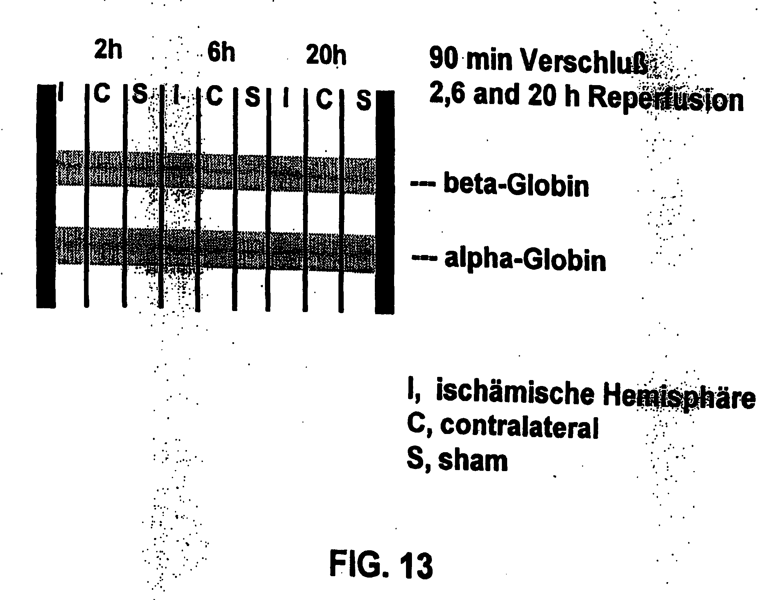

Fig. 13 zeigt einen RMDD-Bandenausschnitt mit Darstellung von alpha- und beta-Globin. Gezeigt sind die primär identifizierten Banden, die als alpha- und beta-Globin identifiziert werden konnten, aus Maushimen nach MCAO mit Reperfusion 2, 6 und 20 h. Aufgetragen sind jeweils die ischämische Hemisphere, die contralaterale Hemispher, und sham-operierte Tiere.

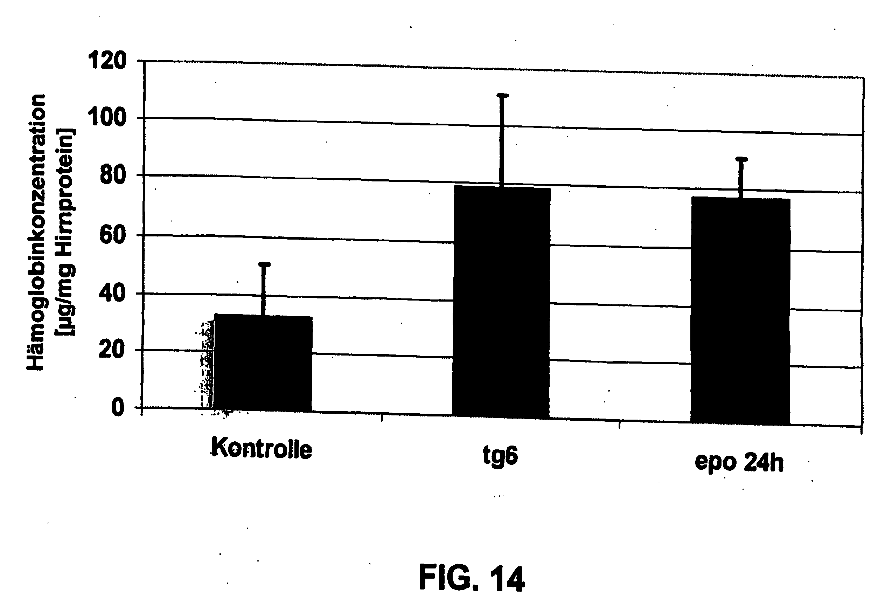

Fig. 14 zeigt in Form eines Balkendiagramms die Ergebnisse eines Versuchs zur Messung des Proteinanteils von Hämoglobin am Gesamtprotein in Hirnproben nach intraperitonealer Erythropoetin-Injektion.

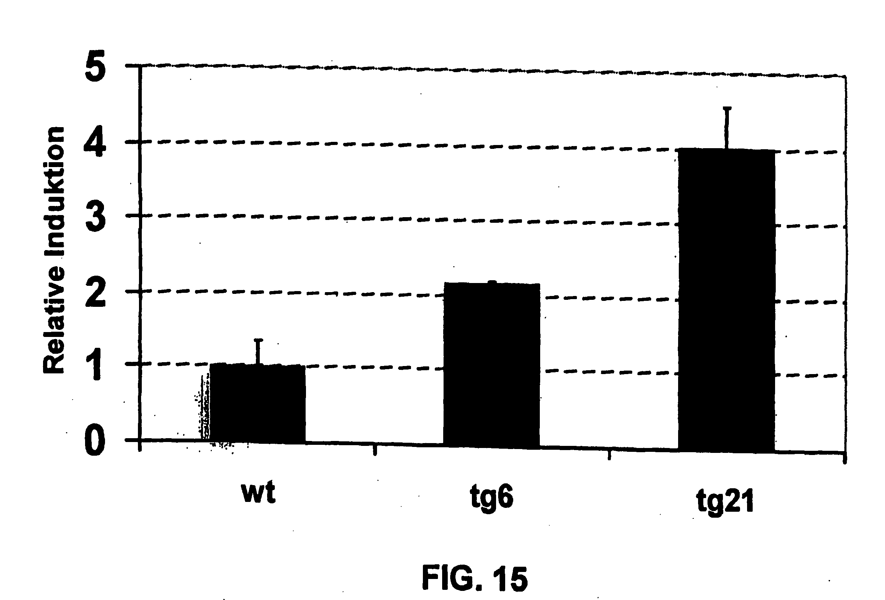

Fig. 15 zeigt das Ergebnis einer quantitativen PCR für DALDH in Wildtyp (wt) sowie EPO-transgenen Tieren.

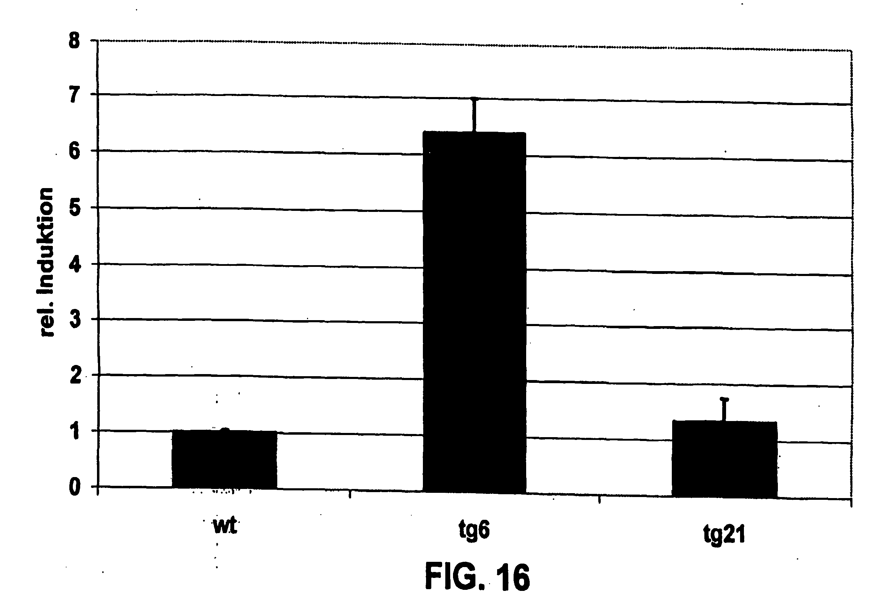

Fig. 16 zeigt das Ergebnis einer quantitativen PCR für ALAS in Wildtyp (wt) sowie EPO-transgenen Tieren.

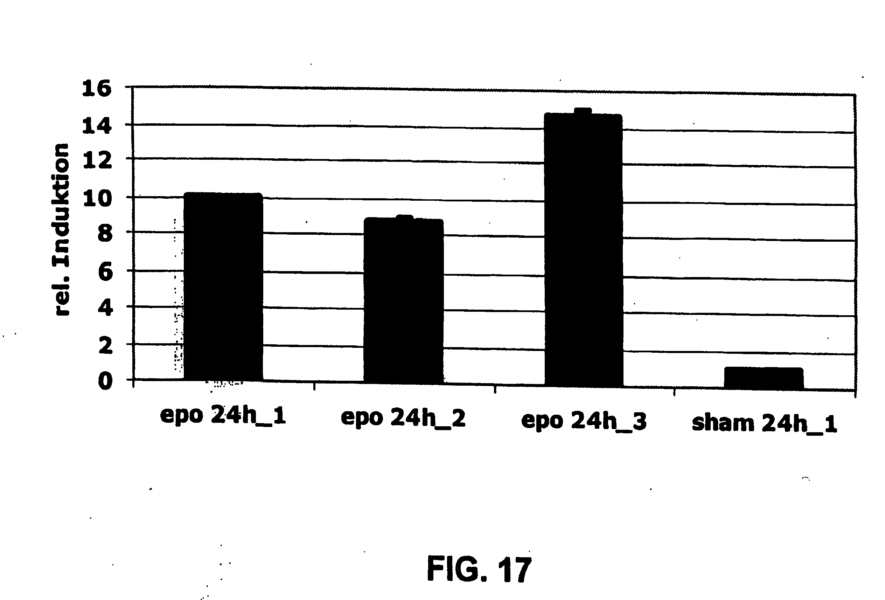

Fig. 17 zeigt das Ergebnis einer quantitativen PCR für ALAS in EPO-injizierten Tieren nach 24 Stunden.

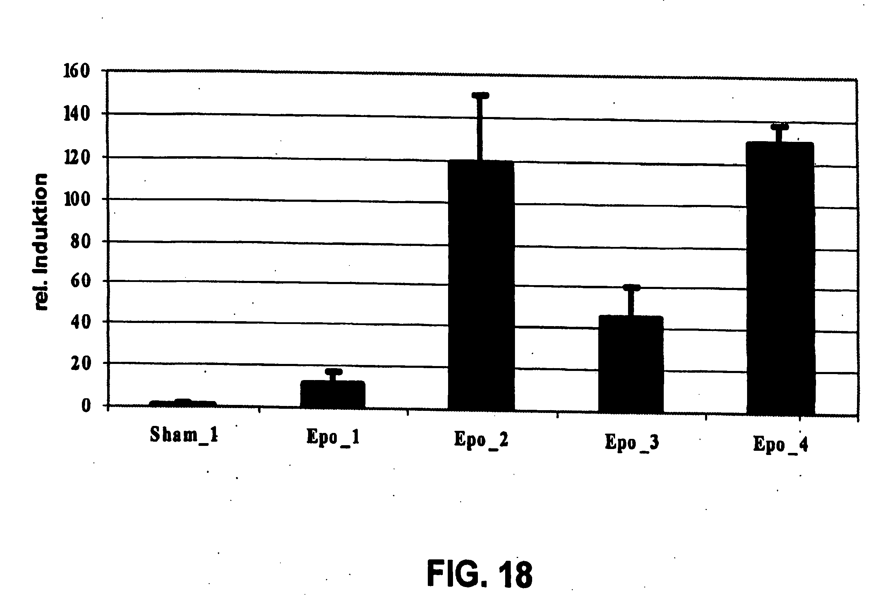

Fig. 18 zeigt das Ergebnis einer quantitativen PCR für ALAS in EPO-injizierten Tieren nach 7 Tagen.

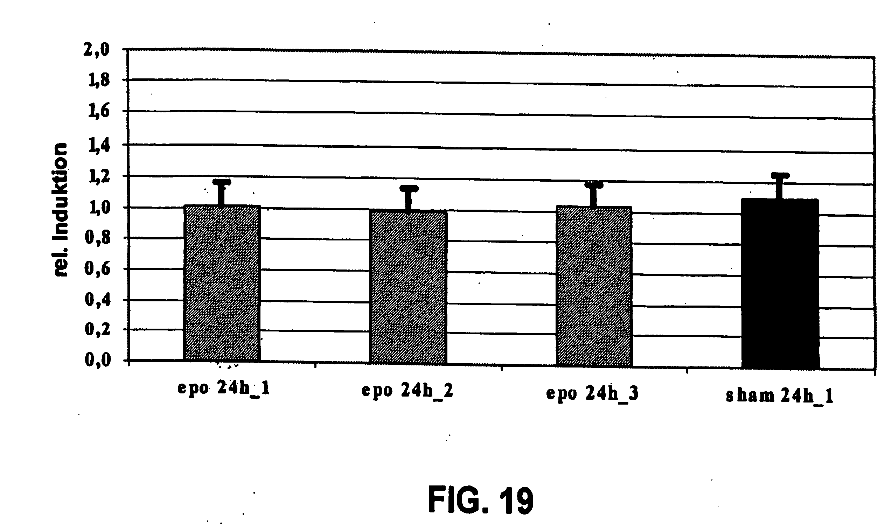

Fig. 19 zeigt das Ergebnis einer quantitativen PCR für Neuroglobin nach Erythropoetin-Injektion.

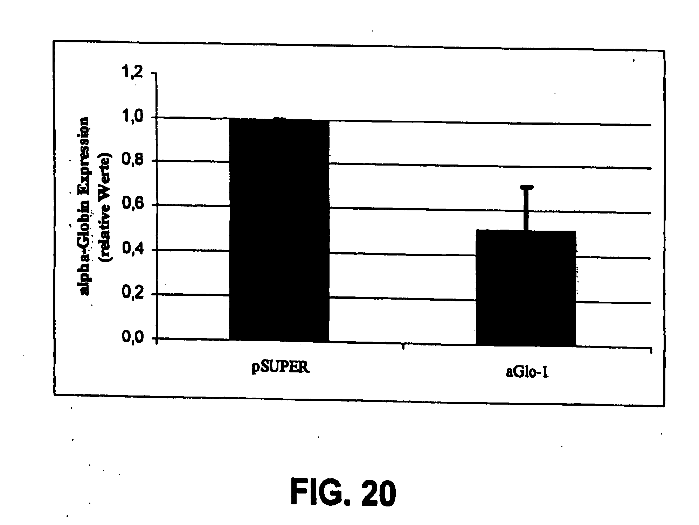

Fig. 20 zeigt das Ergebnis einer quantitativen PCR für alpha-Globin-mRNA zur Bestimmung der Effektivität des siRNA-Konstruktes pSaGlo1 in transient transfizierten PC12 Zellen.

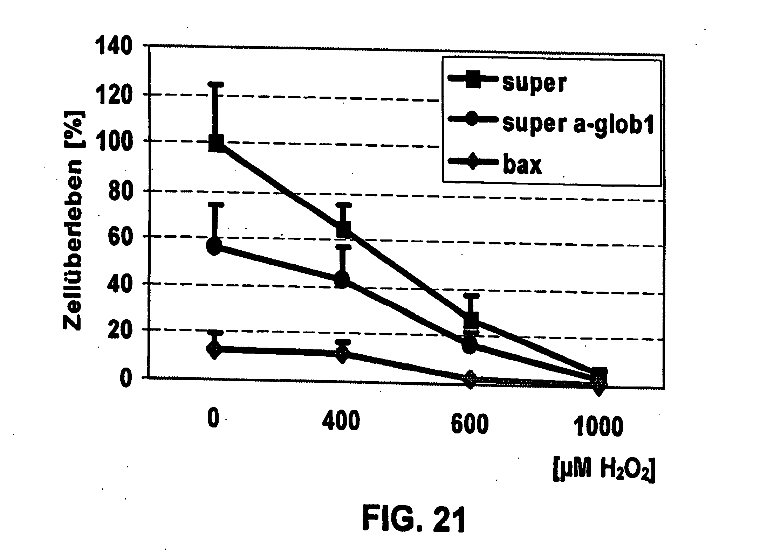

Fig. 21 zeigt das Ergebnis einer Messung zur Bestimmung des Effekts von alpha-Globin-mRNA auf die Überlebensfähigkeit von Zellen. Gemessen wurde das Zellüberleben in % nach der Transfektion von PC12-Zellen mit dem siRNA-Konstrukt pSaGlo1.

### BEISPIELE

### Beispiel 1: Identifizierung von neuronalem alpha- und beta-Globin als Erythropoetinregulierte Gene

Transgene polyglobule Mäuse, die humanes Erythropoietin überexprimieren, wurden wie beschrieben hergestellt (Linien tg6 und tg21: Ruschitzka et al., 2000, Proc Natl Acad Sci USA 97,11609-13; Wagner et al., 2001, Blood 97, 536-42; Wiessner et al., 2001, J Cereb Blood Flow Metab 21,857-64). C57BL6 Wildtyp-Geschwistertiere dienten als Kontrolle.

Die Mäuse wurden mit einem rekombinanten Konstrukt nach Verfahren hergestellt, die dem Fachmann geläufig sind. Dieses Konstrukt bestand aus einem PDGF-Promotor, der eine Expression des rekombinanten EPO vor allem in neuronalen Zellen bewirkt (Sasahara et al., 1991, Cell 64, 217-27), und der kodierenden Sequenz für Erythropoetin. Dabei entstanden mehrere transgene Linien, von denen tg6 und tg21 untersucht wurden. Nur tg6 wies eine systemisch erhöhte EPO-Expression auf, was durch Serumuntersuchungen belegt wurde (Ruschitzka, et al.,2000; a.a.O.). Die Linie tg21 wies keine erhöhten systemischen EPO-Level aus.

Bei den Mäusen der Linie tg6 führte die erhöhte systemische Expression von EPO zu einer deutlichen Steigerung der Erythropoese mit der Folge einer Polyglobulie bis zu einem Hämatokrit von 0,8 und einem deutlich gesteigerten Blutvolumen (bis 4,0 ml). Invivo-Untersuchungen zeigten bei der tg6-Linie einen global erhöhten Hirnstoffwechsel (cerebral glucose utilization). Ursachen dafür könnten entweder eine generalisiert erhöhte neuronale Aktivität oder eine chronische Hypoxie sein. Für eine generalisiert erhöhte neuronale Aktivität gab es keine Anzeichen wie z. B. Krampfanfälle oder hohe motorische Aktivität. Deshalb sprechen die Befunde für eine chronische Hypoxie. In dieselbe Richtung weist auch die generalisiert erniedrigte Hirndurchblutung bei diesen Mäusen: Eine verminderte Himdurchblutung (cerebral blood flow) führt ebenfalls zu einer Gewebshypoxie (Frietsch et al., 2001, JCBF 21, Suppl 1, S.17). Die Linie tg21 weist demgegenüber keine starken phänotypischen Auffälligkeiten auf.

Zur Entnahme des Gehirns wurden die Mäuse mit 0,8 % Isofluran, 70 % N₂O und 29 % Sauerstoff betäubt. Um auszuschließen, dass das in den Versuchen gefundene Hämoglobin aus dem Blut stammt, wurden die Versuchstiere mit einkalter Kochsalzlösung perfundiert, um das Blut aus den Blutgefässen auszuwaschen.

Das Gehirn der transgenen Erythropoietin-überexprimierenden Mäuse wurde unter Narkose nach transkardialer Perfusion entnommen und in flüssigem Stickstoff schockgefroren. RNA wurde nach der Methode von Chornczynski und Sacchi (1987; Anal. Biochem. 162, 156-159) gewonnen. Anschließend wurde ein Hybridisierungsexperiment mit 2 transgenen und 2 Littermate-Kontrollen auf einem Maus-cDNA-Array ("Chip") durchgeführt (Fig. 1). Das Hybridisierungsexperiment ergab eine Reihe von hochregulierten Sequenzen. Dabei fiel eine Sequenz auf, die in den Tieren sowohl der Linie tg6 wie auch der Linie tg21 hochreguliert schien. Diese Sequenz konnte durch Durchsuchen öffentlicher Datenbanken als alpha-Globin identifiziert werden.

### Beispiel 2: Nachweis erhöhter Hämoglobin-Boten-RNA Im Gehirn Erythropoietinüberexprlmierender transgener Mäuse

Zur Messung der Hämoglobin-Boten-RNA wurde die semiquantitative RT-PCR auf dem UghtCyder (Roche, Mannheim, Germany) eingesetzt. Die Quantifizierung erfolgte durch den Vergleich der relativen Fluoreszenz der Probe mit einer Standardkurve für Cyclophilin.

Gesamt-RNA wurde aus Maushim isoliert mit dem RNeasy-Extraktionskit gemäss den Anweisungen des Herstellers (Qiagen, Santa Clarita, CA, USA). Die RNA-Konzentration wurde photometrisch bestimmt und die Qualität der Gesamt-RNA über Agarose-Geleiektrophorese beurteilt. Die RNA wurde bis zum Gebrauch bei -80 °C aufbewahrt.

Nach reverser Transkription mit Superscript II (Invitrogen-Life Technologies, Carisbad, CA, USA) wurden die Reaktionsprodukte durch Real-time online PCR mittels der LightCyder-Technologie relativ quantifiziert. Dafür wurden Gesamt-RNA Proben aus dem Gehirn von drei Wildtyp-Mäusen und drei transgenen tg6-Mäusen eingesetzt. Die spezifischen Sequenzen der Oligonukleotid-Primer lauteten für Cyclophilin
5'-ACCCCACCGTGTTCTTCGAC-3'
   für den Vorwärts- und
5'-CATTTGCCATGGACAAGATG-3'
   für den Rückwärts-Primer bei einer Bindungstemperatur von 60 °C und für Hämoglobin-alpha
5'-GGTGCCCTGTCTGCTCTG-3'
   für den Vorwärts und
5'-GGCAGCTTAACGGTACTTGG-3'
   für den Rückwärts-Primer bei 55 °C Bindungstemperatur.

Zur Quantifizierung wurden serielle cDNA-Verdünnungen von 1:3, 1:9, 1:27, 1:81 und 1:243 nach folgendem Schema amplifiziert: Initiale Denaturierung 5 min bei 94°C, Amplifizierung über 50 Zyklen bestehend aus 5 s Denaturierung bei 94°C, 10 s Bindung bei 55°C oder 60°C - je nach spezifischen Primer (s. o.) - und 30 s Extension bei 72°C. Am Ende jedes Zyklus wurde die Fluoreszenz jeder Probe bei 80°C für 10 s gemessen. Die Spezifität des Reaktionsproduktes wurde durch Agarose-Gelelekrophorese (Fig. 2) und Schmelzkurvenanalyse (nicht gezeigt) nachgewiesen. Jede PCR-Reaktion brachte genau ein Reaktionsprodukt hervor.

Für die Quantifizierung wurde die logarithmische Phase der PCR-Reaktion benutzt Dabei wird eine Asymptote durch die entsprechende Kurve gelegt Für Hämoglobin ergab sich ein nahezu paralleler Anstieg der Geradenschar, so dass die Steigungen dieser Kurven zum Vergleich mit den Standardkurven für Cyclophilin herangezogen werden konnten. Mittelwerte ± Standardabweichung wurden für jede cDNA-Verdünnung aus dem normalisierten PCR-Produkt bestimmt (Fig. 3). Die so erhaltenen quantitativen Unterschiede zwischen entsprechen relativen Veränderungen der RNA-Expression in transgenen und Wildtyp-Tieren. Alle Reaktionen führten zu einem einzigen Reaktionsprodukt (angezeigt durch einen eizelnen Gipfel der Schmelzkurve bei etwa 90 °C für die transgenen und Wildtyp-Mäuse). Der mittlere Induktionsfaktor betrug für Hämoglobin-alpha 3,86 ± 0,18 (n = 3).

### Beispiel 3: Erhöhung von Hämoglobin-Protein im Gehirn Erythropoletln-überexprlmierender transgener Mäuse

Durch semiquantitative zweidimensionale Gelelektrophorese wurden Veränderungen der Expression von Hämoglobin-alpha und Hämoglobin-beta im Gehirn von sechs transgenen tg6-Mäusen und sechs Wildtyp-Mäusen gemessen.

Für die zweidimensionale Gelelektrophorese wurde das Himgewebe in 2 ml Probenpuffer bestehend aus 40 mmol/l Tris, 7 mol/l Harnstoff. 2 mol/l Thioharnstoff, 4 % (w/v) CHAPS, 10 mmol/l DTT und 1 mmol/l EDTA homogenisiert und für 45 min bei 100.000 x g zentrifugiert. Der Proteingehalt der Proben wurde nach der Bradford-Methode bestimmt (Bradford, 1976, Anal. Biochem. 72, 248-254).

Die zweidimensionale Gelelektrophorese wurde nach Standardprotokollen ausgeführt (Görg et al., 2000, Electrophoresis 21, 1037-1053). Proben von 500 µg wurden zur isoelektrischen Fokussierung auf nicht-lineare pH 3-10 Gradienten-IEF-Gelstreifen aufgetragen (Amersham Bioscience, Freiburg, Germany). Nach 6 h Schwellzeit wurden 30 V für 6 h angelegt, dann 500 V für 1 h und 1000 V für 1 h. Danach wurde die Spannung auf 8000 V erhöht und über 10 h konstant gehalten. Daraus ergaben sich 81680 Vh auf dem IPGphor IEF System für die isoelektrische Fokussierung. Die Trennung in der zweiten Dimension wurde in 12,5 % Polyacrylamid-Gelen in Gegenwart von 10 % SDS vollzogen. An die Gele (180 x 200 x 1,5 mm³) wurden 30 mA für 30 min und 100 mA für etwa 4 h in einer wassergekühlten vertikalen Elektrophoresekammer angelegt (OWL Scientific, Wobum, MA, USA). Die Gele wurden nach einem modifizierten Protokoll (Blum et al., 1987, Electrophoresis 8, 93-99) mit Silbernitrat gefärbt, um die Proteine sichtbar zu machen. Diese Methode ist kompatibel mit einer sich anschließenden Massenspektrometrie.

Die Gele wurden dann eingescannt und die Bilder densitometrisch vermessen mit der speziellen Software Phoretix 2D Professional (Nonlinear Dynamics Ltd., Newcastle-upon-Tyne. UK). Nach einer Hintergrundkorrektur wurden die Proteinpunkte für Hämoglobin-alpha und Hämoglobin-beta nach optischer Dichte und Volumen vermessen. Mittelwerte ± Standardabweichungen wurden von sechs transgenen Tieren mit denen von sechs Wildtyp-Tieren mit dem t-Test nach Student verglichen. Ein p-Wert < 0,05 wurde als Signfikanzgrenze angenommen. Die Proteine wurden durch Massenspektrometrie identifiziert (ZMBH, Center of Molecular Biology Heidelberg, Heidelberg, Germany). Die mittleren Induktionsfaktoren betrugen 2,68 ± 0,29 (n = 6) für Hämoglobin-alpha und 2,59 ± 0,37 (n = 6) für Hämoglobin-beta (Fig. 4).

Zusätzlich wurde mit der Cyanhämiglobin- Schnellmethode (van Kampen and Zijlstra, 1961, Clin. Chim. Acta 6, 538-544) die absolute Hämoglobin-Konzentration im Gehirn von sechs transgenen und sechs Wildtyp-Mäusen gemessen. Dazu wurden 20 µl der entsprechenden Himhomogenat-Proben zu 5 ml einer Transformationslösung bestehend aus 200 mg/l Kaliumhexacyanoferrat, 50 mg/l Kaliumcyanid, 140 mg/l Kaliumdihydrogenphosphat und 0,05 % (v/v) Triton X-100 zugegeben und mindestens 5 min bei Raumtemperatur inkubiert. Dann wurde die Extinktion bei 546 nm gemessen und mit 36,7 multipliziert um die Konzentration in g/dl zu erhalten. Die Konzentrationen betrugen 0,029 ± 0.012 mg/(dl Himhomogenat) (n = 6) für die transgenen und 0,011 ± 0,006 mg/(dl Himhomogenat) (n = 6) für die Wildtyp-Gruppe (Fig. 5).

### Beispiel 4: Lokalisierung von Hämoglobin-Boten-RNA im Gehirn Erythropoietinüberexprimierender transgener Mäuse

Die Expressionsorte für Hämoglobin-alpha Boten-RNA wurden durch in-situ Hybridisierung mit Digoxigenin-markierten Oligonukleotiden sichtbar gemacht. Spezifische Bindungen wurden in Maushimgefrierschnitten durch Inkubation mit einem Anti-Digoxigenin-Antikörper sichtbar gemacht, an den alkalische Phosphatase gebunden ist.

Die in-situ Hybridisierung wurde durchgeführt wie für Digoxigenin-markierte Oligonukleotidsonden beschrieben (Wisden and Morris, 1994, In situ hybridization protocols for the brain, Academic Press, London; San Diego). Dazu werden 10 µm dicke Kryoschnitte in eiskaltem Paraformaldehyd 5 min fixiert, 5 min in 1 X PBS (130 mM NaCl, 7 mM Na₂HPO₃, 3 mM NaH₂PO₃) gewaschen, 5 min in 70 % (v/v) Ethanol dehydriert und in 96 % (v/v) Ethanol aufbewahrt. Die Hybridisierungssonde für Hämoglobin hatte die Sequenz
5'-CTTACATCAAAGTGAGGGAAGTAGGT-3'
und war am 3'-Ende mit Digoxigenin markiert (MWG-Biotech, Ebersberg, Germany). Die Sonde wurde verdünnt in Hybridisierungspuffer bestehend aus 5 g Dextransulfat gelöst in 25 ml ionenfreiem Formamid. 10 ml 20 X SSC (3 M NaCl, 0,3 M Natriumcitrat, pH 7,0) und sterilem, RNase-freiem Wasser auf 50 ml. Dies ergab eine Endkonzentration von 30 ng Sonde pro Schnitt. Hybridisiert wurde bei 42°C über Nacht im Wasserbad in einer Inkubationskammer, die mit 1:1 Formamide zu 4 X SSC feucht gehalten wurde. Die Schnitte wurden am nächsten Tag in 1 X SSC für 10 min bei Raumtemperatur (RT), 1 X SSC für 30 min bei 60°C,1 X SSC für 1 min bei RT und 0,1 X SSC für 1 min bei RT gewaschen.

Die Schnitte wurden dann zwei Mal 10 min lang in Detektions-und-Wasch-Puffer (150 mM NaCl, 100 mM Tris-HCl, pH 7,5) inkubiert und unspezifische Antikörperbindungen wurden durch 2 % (v/v) normales Schafserum in Detektions-und-Wasch-Puffer mit 0,1 % (v/v) Triton X-100 für 30 min blockiert Danach wurden die Schnitte 4 h lang in einer Anti-Digoxigenin-Antikörper-Lösung inkubiert (Roche, Mannheim, Germany, 1:200 in Blocklösung). Die Schnitte wurden zwei Mal 10 min lang in Detektions-und-Wasch-Puffer gewaschen und der pH-Wert durch Eintauchen für 10 min in Substratpuffer (100 mM NaCl, 50 mM MgCl₂. 100 mM Tris-HCl, pH 9,5) abgeglichen. Die Farbreaktion wurde mit BCIP/NBT für 2-24 hours soweit entwickelt, bis eine ausreichende Färbung aufgetreten war. Die Reaktion wurde mit 10 mM Tris-HCl, pH 8,1, 1 mM EDTA gestoppt und die Schnitte zur Lichtmikroskopie eingebettet Für die Kontrollexperimente wurden die Schnitte entweder ohne Oligonukleotid oder ohne Antikörper inkubiert.

Gebundene Antikörper produzierten ein Farbsignal mit BCIP/NBT als Substrate im Hippocampus und dem Kleinhirn der Versuchstiere, wobei die Signale in den transgenen Tieren stärker waren als in den Wildtyp-Tieren. Kontrollexperimente ohne Oligonukleotid und ohne Antikörper zeigten nur eine normale Hintergrundfärbung (Fig. 6). Es findet sich prominente Expression in der Kömerzellschicht des Cerebellums, im Gyrus dentatus, in allen Arealen des Hippocampus, in den Habenulae und deren Commissur. Daneben sieht man schwächere verteilte Signale im Cortex, Striatum und Thalamus, die mit neuronaler Expression vereinbar ist. Keine Signale finden sich im Corpus callosum, und anderen Bereichen mit weisser Substanz.

### Beispiel 5: Lokalisierung von Hämogiobln-Protein im Gehirn Erythropoietin-überexprimierender transgener Mäuse

Hämoglobin-Protein wurde durch Anfärbung von Kryoschnitten mit 2,7-Diaminofluoren (DAF) in der Dunkelfeldmikroskopie sichtbar gemacht. Ein Protokoll für die DAF-Färbung wurde für 10 µm Kryoschnitte adaptiert (Worthington et al., 1987, Exp. Hematol. 15, 85-92): Die Schnitte wurden 2 min lang in eiskaltem Azeton fixiert und luftgetrocknet. Die Zellmembranen wurden durch dreimaliges Waschen in 200 mM Tris-HCl, pH 7,0, mit 0,2 % Tween-20 permeabilisiert und die Schnitte für 15 min bei Raumtemperatur in einer Mischung von 100 µl 1 % (w/v) DAF in 90 % Eisessig, 100 µl 30 % (w/v) H₂O₂ und 10 ml 200 mM Tris-HCl, pH 7,0 inkubiert. Anschließend wurden die Schnitte im Dunkelfeldmikroskop untersucht.

Eine deutliche Anfärbung trat auf im Hippocampus, dem Kleinhim-Cortex und dem paraventrikulären Kern des Thalamus (Fig. 7). Die Expressionsorte für das Hämoglobin-Protein korrelieren mir den Expressionsorten für die Hamogiobin-Boten-RNA. Auf DAF-gefärbten Schnitten des Hippocampus von Wildtyp- (A) und transgenen (B) Tieren (Fig. 8) zeigt sich bei letzteren ein deutlich stärkeres und spezifischeres Signal.

Um die Färbung zu optimieren wurden, zusätzliche Experimente durchgeführt bei denen die Inkubationszeit in DAF-Puffer (1 % (w/v) DAF in 90% Eisessig, 100 µl 30 % (w/v) H₂O₂ und 10 mL 200 mM Tris-HCl, pH 7.0) von 15min auf 60min erhöht Die Figuren 7.1 und 8.1 zeigen nun deutlicher die Lokalisation von Hämoglobin beziehungsweise die Unterschiede zwischen Wildtyp- und EPO-transgener Maus.

### Beispiel 6: Nachweis der direkten Induktion von Hämoglobin durch Erythropoetin

Die Induktion von Hämoglobinexpression in Mäusen, die transgen Erythropoetin überexprimieren, kann prinzipiell auf verschiedene Effekte von Erythropoetin zurückgeführt werden. Es könnte sein, dass die Überexpression von EPO in frühen Entwicklungsstadien der Maus dazu führt, dass es aufgrund eines veränderten Entwicklungsprogrammes der Neuronen, z.B. der hippokampalen Neuronen, zu einer konstitutiven Mehrexpression von Hämoglobin kommt. Ebenso könnte es sein, dass nur die langfristige (monatelange) Überexpression von EPO diesen Effekt hervorruft. z.B. über komplexere zwischengeschaltete indirekte Mechanismen. Um die direkte Wirkung von EPO auf die neuronale Hämogiobirtexpression zu zeigen, wurde den Ratten intraperitoneal und intracerebral Erythropoetin verabreicht und die mRNA-Expression von alpha- und beta-Globin im Gehirn mittels quantitativer PCR 6 oder 24 h nach Gabe nachgewiesen. Dabei wurden folgende Dosen verwendet: 50 µl EPO-Lösung in den dorsalen Hippocampusbereich (etwa im Bereich der CA3 Subregion) mit einer Dosis von 25 units (U). Dies wurde für 4 Ratten durchgeführt, 2 der Ratten wurden nach 6 h, 2 nach 24 h getötet Ebenso wurden sham-behandelte Tiere präpariert.

RNA wurde aus den Himhemisphären und aus dem Kleinhirn gewonnen, jeweils für die rechte und linke Hemisphere. Die Injektion erfolgte in die rechte Hemisphäre mittels eines Stereotakten. Der Injektionsort wurde mit Hilfe von Evans-Blue Injektionen verifiziert. Für die intraperitoneale Injektion wurde eine Dosis von 5000 U/kg verabreicht; dies geschah ebenfalls für je 2 Tiere, und für die Zeitpunkte 6 und 24 h. Die Zeitpunkte wurden aufgrund der Wahrscheinlichkeit des Auffindens einer mRNA Regulation durch direkte EPO-Einwirkung gewählt. Auch hier wurden entsprechende sham-injizierte Tiere präpariert

Nach Gewinnung der RNA (Chomzynski und Sacchi, a.a.O.) gefolgt von Aufreinigung über Qiagen RNA_easy Säulen nach Herstellerangaben wurde cDNA mit Hilfe von oligo-dT-Primem und Superscript II-Reverser Transkriptase gewonnen, gefolgt von Aufreinigung über Qiagen-Saulchen (cDNA Aufreinigungsprotokoll). Quantitative PCR wurde mit Hilfe des LightCyder-Systems (Roche, Mannheim, Germany) durchgeführt, wie schon oben beschrieben. Alpha- und beta-Globin wurden relativ gegen Cyclophilin quantifiziert. Die verwendeten Primer waren:
alpha globin: Primer rm_a_glob_s: 5'-GGGAAGATTGGTGGCCATGGTG-3', Tm 59°
   Primer rm_a_glob_a: 5'-GGCAAGGAATTTGTCCAGAGAGGC-3', Tm 59°
beta globin: Primer rm_b_glob_s: 5'-CTGACTGATGCTGAGAAGGCTGCT-3' Tm 59°
   Primer rm_b_glob_a: 5'-TCCAGCCACCACCTTCTGGAAG-3', Tm = 59°
Cyclophilin: 5'-ACCCCACCGTGTTCTTCGAC-3' für den Vorwärts- und
   5'-CATTTGCCATGGACAAGATG-3' für den Rückwärtsprimer.

Es ergab sich jeweils eine signifikante Erhöhung von alpha- und beta-Globin in den intraperitoneal behandelten Mäusen, die jeweils im Bereich von 2-4 fach lagen (siehe Fig. 9 und 10). Dieses Ergebnis belegt deutlich, dass der Effekt von EPO auf die Hämoglobinexpression zumindest zum grössten Tell kein Entwicklungsphänotyp ist, und dass der Effekt kurzfistig wirksam, und deshalb wahrscheinlich direkt ist.

### Beispiel 7: Identifizierung von alpha- und beta-Globin als Ischämie-induzierte Genprodukte

Das Tiermodell der fokalen cerebralen Ischämie (middle cerebral artery occlusion, MCAO) stellt ein Modell für den humanen ischämischen Schlaganfall dar. Zur Herbeiführung der fokalen cerebralen Ischämie wurde das sog. Fadenmodell benutzt, bei dem ein beschichteter Nylonfaden durch die A. carotis interna an den Abgang der A. cerebri media vorgeschoben wird und einen ischämischen Schlaganfall induziert (Clark et al.,1997,Neurol. Res. 19.641-648).

Zum Herbeiführen einer fokalen cerebralen Ischämie in c57/b16-Mäusen (Fa. Charles-River WIGA, Sulzfeld, Deutschland) wurden 3 Monate alte Mäuse benutzt. Nach Herbeiführen einer Inhalationsnarkose (70 % N₂0, 30 % O₂, 0.8-1 % Halothan) wurde ein 5-0-Prolenefaden (Fa. Ethicon, Norderstedt), der mit 0,1 % Poly-L-Lysin beschichtet war, über die A. carotis externa in die A. carotis interna bis zum Abgang der A. cerebri media vorgeschoben. Die richtige Position des Fadens wird durch einen Abfall des Laser-Doppler-Signals (Fa. Perimed, JärfÄlla, Schweden) auf 10-20 % des Ausgangssignals angezeigt Nach Durchführung dieser Operation und gegebenenfalls Bestimmung zusätzlicher physiologischer Parameter (Blutdruck, Puls, Blutgase, Blutglukose) erwachen die Mäuse aus der Narkose. Nach bestimmten Okklusionszeiten werden die Mäuse wieder einer Narkose unterzogen, und der Faden zurückgezogen. Dadurch findet eine Reperfusion des Gewebes statt. Nach bestimmten Reperfusionszeiten werden die Mäuse durch Genickbruch getötet. Das Gehirn wird sofort präpariert und auf Trockeneis weggefroren. Im vorliegenden Fall wurden Okklusionen für 90 min, und Reperfusionen für 2 h, 6 h, und 20 h durchgeführt.

### 1) Präparation von mRNA aus den Hirnen und Nachweis von Hämoglobin-Boten-RNA

Dazu wurde nach der Methode von Chomczynski und Sacchi (a.a.O.) verfahren, gefolgt von einer weiteren Aufreinigung mit Hilfe von RNA-Aufreinigungssäulchen (RNAeasy, Qiagen) nach Angaben des Herstellers.

Für die Quantifizierung der mRNA-Regulation durch cerebrale Ischämie (MCAO-Modell) wurde wie in Beispiel 2 verfahren, es wurde jedoch hier zusätzlich beta-Globin bestimmt. Die benutzten Primer haben folgende Sequenz (identisch für Maus und Ratte):
Alpha-Globin: Primer rm_a_glob_s: 5'-GGGAAGATTGGTGGCCATGGTG-3', Tm 59° und
   Primer rm_a_glob_a:5'-GGCAAGGAATTTGTCCAGAGAGGC-3'. Tm 59°
Beta-Globin: Primer rm_b_lglob_s: 5'-CTGACTGATGCTGAGAAGGCTGCT-3'. Tm = 59°
   Primer rm_b_glob_a: 5'-TCCAGCCACCACCTTCTGGAAG-3', Tm = 59°

Es ergaben sich Induktionsfaktoren (ischamische gegen kontralaterale Hemisphere) für alpha-Globin von 2,41 ± 0,07 und 1,78 ± 0,24 in zwei Tieren mit MCAO und 20 h Reperfusion (Fig. 11). Für beta-Globin ergaben sich Induktionsfaktoren von 3,56 ± 0,12 in einem Tier und 1,97±0,37 in einem zweiten (Fig. 12). Im Vergleich zu sham-operierten Tieren ergaben sich vergleichbare Werte.

### 2) Durchführung des RMDD(restriction mediated differential display)-Protokolls

Dabei wurde im wesentlichen nach EP 0 743 367 A2 und US 5 876 932 verfahren, mit der Änderung, dass 2 ug polyA-RNA für die Erststrangsynthese eingesetzt wurden. Nach Durchführung von Erststrangsynthese, Zweitstrangsynthese und Restriktionsverdau wird eine Adapterligation mit Adaptoren durchgeführt. Nach erfolgter PCR-Amplifikation mit Adapter- und cDNA-Primem werden die Amplifikationsprodukte auf ein denaturierendes Gel geladen und auf eine Nylonmembran geblottet (Fa. GATC. Konstanz, Deutschland). Die Biotin-markierten Banden werden mit Hilfe einer gebräuchlichen Streptavidin-Peroxidase-Reaktion (Roche Molecular Biochemicals, Mannheim, Deutschland) visualisiert. Auf das Gel wurden jeweils PCR-Proben von der ischämischen und kontralateralen Hemisphäre zusammen aufgetragen (24 h MCAO rechts und links und 90 min MCAO rechts und links). Banden unterschiedlicher Intensität in der rechten oder linken Hemisphäre werden ausgeschnitten, und eine Reamplifikation des entsprechenden PCR-Produktes durchgeführt Erhaltene amplifizierte Produkte werden in den TOPO TA Vektor pcDNA 2.1 (Fa. Invitrogen, Carisbad, CA, USA) kioniert und mit T7 und M13rev-Primern sequenziert (ABI 3700 Kapillarelektrophoresesequencer, Fa. PE Applied Biosystems, Foster City, CA, USA). Erhaltene Sequenzen werden mit der EMBL-Datenbank verglichen. Dabei ergaben sich Sequenzen für alpha- und beta-Globin (Fig. 13).

### Beispiel 8: Nachweis direkter Hämoglobininduktion im Gehirn durch einmalige Erythropoetin-injektion (intraperitoneal)

In Fortführung von Beispiel 3 wurde die Hämoglobinkonzentration ebenfalls im Him von Mäusen bestimmt, denen intraperitoenal Erythropoetin (Erypo der Fa. Janssen) appliziert wurde (5000 units/kg Körpergewicht). Nach Injektion von EPO wurden die Mäuse nach 24 h durch Injektion mit Rompun/Ketanest terminal anaesthesiert, und durch transkardiale Perfusion mit 40 ml Hanks balanced salt solution (HBSS) wurde das Gefäßsystem im Gehirn von Blut freigespült Dazu wurde ein Himhomogenat hergestellt in 2 ml Probenpuffer bestehend aus 40 mmol/l Tris, 7 mol/l Harnstoff, 2 mol/l Thioharnstoff, 4 % (w/v) CHAPS, 10 mmol/l DTT und 1 mmol/l EDTA homogenisiert und für 45 min bei 100.000 x g zentrifugiert. Der Proteingehalt der Proben wurde nach der Bradford-Methode bestimmt (Bradford, 1976, Anal. Biochem. 72, 248-254). Die Hämoglobinkonzentration wurde mit der Cyanhämiglobin- Schnellmethode (van Kampen and Zijlstra, 1961. Clin. Chim. Acta 6, 538-544) gemessen. Dann wurden 20 µl der entsprechenden Himhomogenat-Proben zu 5 ml einer Transformationslösung bestehend aus 200 mg/l Kaliumhexacyanoferrat, 50 mg/l Kaliumcyanid, 140 mg/l Kaliumdihydrogenphosphat und 0,05 % (v/v) Triton X-100 zugegeben und mindestens 5 min bei Raumtemperatur inkubiert Dann wurde die Extinktion bei 546 nm gemessen und mit 36,7 multipliziert um die Konzentration in g/dl zu erhalten. Diese Werte wurden dann auf die Proteinkonzentration / Volumen Himgewebe bezogen, und es ergaben sich folgende Werte (unter Einbeziehung der gemessenen transgenen Tiere aus Beispiel 3): Kontrolltiere: 32,54±17,51 µg/mg Himprotein (n =10); EPO-transgene Tiere (Linie tg6): 79,00 ±32,12 (n = 13); EPO-injizierte Tiere (24 h nach Injektion): 77,14±13,51 µg/mg Himprotein (n=3). Der Unterschied war signifikant zwischen Kontrolle und EPO-behandelten Tieren bzw. EPO-transgenen Tieren (ANOVA und post-hoc Test). (Fig. 14). Diese Daten zeigen, dass eine einzige EPO-Injektion, von der neuroprotektive Wirkung gezeigt ist, bereits zu einer messbaren Erhöhung der Konzentration von intaktem Hämoglobin im Him führt Dies unterstreicht die funktionelle Relevanz der Globin-Induktion durch EPO im zentralen Nervensystem.

### Beispiel 9: Induktion von Schlüsselenzymen der Häm-Synthese durch Erythropoetin im Gehirn

Wir konnten in den Beispielen 3 und 5 und 8 nachweisen, dass intaktes Hämoglobin im Gehirn von Neuronen gebildet wird, und von Erythropoetin induziert wird. Die Beispiele 2,4, und 6 belegen, dass EPO direkt die Menge an Boten RNA für alpha und beta Globin erhöht Hämoglobin enthält jedoch neben den Proteinanteilen a und β Globin (beim Erwachsenen) noch die prostethische Gruppe Härn. Härn ist in Neuronen vorhanden, und kann von der Hämoxygenase abgebaut werden.
Die Synthese von Härn ist genau bekannt Die Synthese beginnt mit der Kondensation von Glycin und Succinyl-CoA zu δ-Aminolävulinsäure. Das Enzym, das diesen Schritt katalysiert ist die δ-Aminolävulinsäuresynthase (ALAS). Diese Synthese ist üblicherweise der wichtige "rate-limiting step" in der Häm-Synthese. Im nächsten Schritt katalysiert die Porphobilinogen Synthase, die auch Aminolävulinsäuredehydratase genannt wird (abgekürzt DALDH) die Kondensation von zwei Molekülen δ-Aminolävulinsäure zu Porphobilinogen (PBG).
Wir fanden erstaunlicherweise, dass die mRNA für DALDH auf dem DNA array (siehe Beispiel 1) eine Induktion auswies (Daten nicht gezeigt). Wir verifizierten die Hochregulation daraufhin für DALDH und ALAS mithilfe der quantitativen PCR im UghtCycter (Roche). Die benutzten Primersequenzen und PCR-Bedingungen waren für ALAS:
alas_m_LC_1s: ACAGACCTGCTGAGCACCATG;
alas_m_LC_1as: AGCCAGCTCCTGTTCAAGCTC
(Annealing Temperatur 60°C ; Messtemperatur 83°C; 45 Zyklen), und für DALDH: daldh_m_1 s:ccaactattgaggctgtccgt, daldh_M_1as:catgagcatgtcagctccttc (Annealing Temperatur. 60°C ; Messtemperatur: 85°C; 45 Zyklen).
DALDH wies in den EPO-überexprimierenden Tieren der Linien tg6 und tg21 eine erhöhte Expression auf: Wildtyp (wt) 1±0,35, tg6 2,18±0,02, tg21 4,02±0,55 (Fig. 15).
ALAS wies in den EPO-überexprimierenden Tieren der Linien tg6 und tg21 ebenfalls eine erhöhte Expression auf: Wildtyp (wt) 1±0,04, tg6 6,39±0,60, tg21 1,33±0,42 (Fig 16).
Darüber hinaus konnten wir für ALAS nachweisen, dass es ebenfalls auf mRNA Ebene direkt durch Injektion einer einmaligen Dosis EPO (5000 units/kg Körpergewicht i.p.) induziert werden kann: sham (sham 24h_1): 1.00±0.08; EPO-injiziertes Tier 1 (epo 24h_1): 10,07±0,00; EPO-injiziertes Tier 2 (epo 24h_2): 8,76±0,18; EPO-injiziertes Tier 3 (epo 24h_3): 14,69±0,27; Fig 17. Durch tägliche Gabe dieser Dosis EPO für 7 Tage konnten Induktionsfaktoren bis 130-fach erzielt werden: sham: 1±0,64; EPO-injiziertes Tier 1 (po_1) = 11,67±5,46; EPO-injiziertes Tier 2 (Epo_2) = 118,93±31,02, EPO-injiziertes Tier 3 (Epo_3) = 44,88±15,15; EPO-injiziertes Tier 4 (Epo_4) = 129,47±8,77 (Fig. 18).

### Beispiel 10: Neuroglobin wird nicht durch eine einmalige Erythropoetin-Injektion Induziert

Neuroglobin ist ein Protein, das geringe Homologie zu Globinen aufweist, und ebenfalls im zentralen Nervensystem gefunden wurde ( Trent, et al. (2001). Journal of Biological Chemistry, 276, 30106-10. ). Allerdings weist es wichtige biochemische Eigenschaften auf, die es grundlegend von den Globinen unterscheidet, z.B. eine vielfach höhere Sauerstoffaffinität ( Dewilde, et al. (2001), Journal of Biological Chemistry, 276, 38949-55.). Neuroglobin ist neuroprotektiv, obwohl der Mechanismus hier unbekannt ist ( Sun, et al. (2001), Proc Natl Acad Sci U S A, 98, 15306-11. , Venis (2001), Lancet, 358, 2055. , Sun, et al. (2003), Proc Natl Acad Sci U S A, 5, 5 ). Um nachzuweisen, ob Neuroglobin möglicherweise ebenfalls eine Rolle in der Erythropoetin-vermittelten Neuroprotektion hat, wurde der relative Gehalt an Neuroglobin mRNA nach EPO Gabe mittels der relativen quantitativen PCR im Vergleich zu Cyclophilin bestimmt. Dabei wurden 5000 units EPO / kg Körpergewicht i.p. appliziert, und die Tiere nach transkardialer Perfusion getötet RNA und cDNA wurde nach Standardverfahren gewonnen. Aus seriellen Verdünnungen der cDNA (3-fach serielle Verdünnungen) wurde jeweils der Gehalt an Cyclophilin und Neuroglobin bestimmt, und das Verhältnis Neuroglobin / Cyclophilin in den shambehandelten Tieren 1 gesetzt Die Standardabweichungen der folgenden Werte beziehen sich auf Messungen bei verschiedenen Verdünnungen (n=4), und repräsentieren die Messgenauigkeit Die benutzten Primer waren: ngl_m_LC_1s:gagtcagagctgatccggcag, ngl_m_LC_1as:caggcacttctccagcatgta für Neuroglobin. Die Primer für Cyclophilin waren: cyc5, ACC CCA CCG TGT TCT TCG AC: acyc300, CAT TTG CCA TGG ACA AGA TG. Als Annealing Temperatur wurde 60°C gewählt, die Messtemperatur für PCR Produkte lag bei 86°C, insgesamt wurden 45 Zyklen durchgeführt Es zeigte sich, dass keine Induktion von Neuroglobin stattfand (sham: 1,10±0,15; EPO-injiziertes Tier 1: 1,01±0,15; EPO-injiziertes Tier 2: 0,99±0,15: EPO-injiziertes Tier 3: 1,04±0,13; Fig. 19), in denselben Tieren, in denen α- und β-globin, sowie δ-Aminolävulinsäuresynthase (ALAS) zu diesem Zeitpunkt stark induziert waren (α-globin ca. 14-fache, β-globin ca. 10-fache, ALAS ca. 10-fache Induktion, siehe auch obige Beispiele). Aus diesen Daten lässt sich folgern, dass Neuroglobin nicht an einer Erythropoetin-vermittelten Neuroprotektion beteiligt ist, die durch Beeinflussung von Genexpression mediiert ist.

### Beispiel 11: Funktionelle Relevanz von intrazellulärem Hämoglobin für neuronale Zellen (PC12 Zellen)

In der Veröffentlichung von Brummelkamp, et al. (2002): Science 19 (296):550-3 wurde ein Vektor beschrieben, der es erlaubt, kurze, doppelsträngige antisense RNA - Moleküle in Zellen zu exprimieren, die zum Abbau der mRNA des spezifischen Zielgens führt (siRNA). Diese Methode sollte angewandt werden, um den neuroprotektiven Effekt des neuronalen Globins zu bestätigen. Zunächst wurde der Vektor pSUPER wie beschrieben hergestellt (Brummelkamp et al., 2002). Anschließend wurden alpha-Globin spezifische DNA-Oligos nach Brummelkamp et al., 2002 designt, synthetisiert (Thermohybaid/ Thermo Biosciences GmbH: aGlo1-s: 5'-p-GATCCCCTTCCTTGCCTCTGTGAGCATTCAAGAGATGCTC ACAGAGGCAAGGAATTTTTGGAAA-3' und aGlo1-as: 5'-p-GCTTTTCCAAAAATTCCTT GCCTCTGTGAGCATCTCTTGAATGCTCACAGAGGCAAGGAAGGG-3') und in einer Konzentration von 10µm in Ligase-Puffer bei zunehmend sinkenden Temperaturen (95°C bis 40°C, über 15min) miteinander hybridisiert Dieses doppelsträngige DNA-Oligomer wurde in den BgIII-HindIII-doppeltverdauten pSUPER ligiert Das so entstandene Konstrukt wurde als pSaGlo1 bezeichnet Dieses Konstrukt und der Leervektor pSUPER wurden jeweils zusammen mit einem Expressionskonstrukt für alpha-Globin und einem Expressionskonstrukt für ein unabhängiges Gen in PC12-Zellen transient transfiziert. Zwei Tage nach der Transfektion wurden die Zellen geerntet und die Gesamt-RNA aus diesen Zellen isoliert Mittels quantitativer RT-PCR in einem LightCycler (Roche Diagnostics, Mannheim, Deutschland) wurde die Effektivität der Herabregulation der alpha-Globin-mRNA durch das siRNA-Konstrukt überprüft. Als Maß für die Transfektionseffizienz diente dabei der mRNA-Level des unabhängigen Gens, der ebenfalls mittels RT-PCR gemessen und als Referenz diente (verwendete Primer Rm_a_glob_1s: 5'-GGGAAGA TTGGTGGCCATGGTG-3'; Rm_a_glob_1es: 5'-GGCAAGGAATTTGTCCAGAGAGGC-3'; Annealing Temperatur: 60°C; Messtemperatur: 85°C; 50 Zyklen) .
In unabhängigen Experimenten konnte gezeigt werden, dass trotz Überexpression des Zielgens die RNA-Menge auf etwa die Hälfte reduziert werden konnte (Fig. 20). Dazu wurden die gemessenen alpha-Globin-Werte in den pSUPER transfizierten Zellen 1 gesetzt (pSUPER = 1±0) und die der pSaGlo1 dazu ins Verhältnis gesetzt (aGlo1 = 0,5±0,2).

In weiteren Versuchen wurde der Effekt von alpha-Globin auf die Überlebensfähigkeit von Zellen getestet
PC12-Zellen wurden in Poly-L-Lysin beschichteten, LIA-96-well Platten mit flachem Boden ausgesät Nach 2 Tagen in Kultur wurden die Zellen mit Renilla-Luciferase-Expressionskonstrukten und pSUPER oder pSaGlo1 oder einem bax-Expressionskönstrukt transfiziert. Nach einem weiteren Tag in Kultur wurden die Zellen H₂O₂ in verschiedenen Konzentrationen ausgesetzt. 20h nach dem H₂O-₂-Stimutus wurde das Überleben der transfizierten Zellen mittels Luciferase-Assay (nach Herstellerangaben: Promega: Renilla Luciferase Assay System; Berthold: Mithras Platereader) bestimmt Eine höhere Luciferase-Aktivität spiegelt eine größere Zahl lebender, transfizierter Zellen wieder.
Zellen in denen die alpha-Globin-mRNA-Expression durch das siRNA-Konstrukt pSaGlo1 herabgesetzt ist (pSUPER aGlob1), reagieren mit Zelltod sensitiver auf H₂O₂ als mit dem Leervektor pSUPER transfizierte Zellen (Fig. 21). Dies deutet auf eine mögliche neuroprotektive Funktion von intrazellulärem Hämoglobin in neuronalen Zellen hin.

## Patentansprüche

1. Screeningverfahren zum Identifizieren von für neuronale Zellen protektive Substanzen, die die Hämoglobinbildung in neuronalen Zellen beeinflussen, umfassend:
a) Inkontaktbringen, in vitro, der zu testenden Substanz mit einer neuronalen Testzelle, und
b) Nachweisen einer veränderten Hämoglobinbildung in der Testzelle.

2. Verfahren gemäß Anspruch 1, **dadurch gekennzeichnet, dass** der Nachweis der veränderten Hämoglobinbildung durch den Nachweis mindestens einer Globin-RNA er- folgt.

3. Verfahren gemäß Anspruch 2, **dadurch gekennzeichnet, dass** die nachzuweisende Globin-RNA alpha-Globin-und/oder beta-Globin-RNA ist.

4. Verfahren gemäß Anspruch 2 oder 3, **dadurch gekennzeichnet, dass** der Nachweis von Globin-RNA mittels Polymerasekettenreaktion (PCR) erfolgt.

5. Verfahren gemäß Anspruch 2 oder 3, **dadurch gekennzeichnet, dass** der Nachweis von Globin-RNA durch Hybridisierung mit einer geeigneten Sonde erfolgt.

6. Verfahren gemäß Anspruch 1, **dadurch gekennzeichnet, dass** der Nachweis der veränderten Hämoglobinbildung durch den Nachweis von Hämoglobin-Protein erfolgt.

7. Verfahren gemäß Anspruch 6, **dadurch gekennzeichnet, dass** der Nachweis von Hämoglobin-Protein durch Färbung mit 2, 7-Diaminofluoren oder mit der Cyanhämiglobin-Methode erfolgt.

8. Verfahren gemäß Anspruch 6, **dadurch gekennzeichnet, dass** der Nachweis der von Hämoglobin-Protein mit einem Antikörper erfolgt.

9. Verfahren gemäß Anspruch 1, **dadurch gekennzeichnet, dass** die Testzelle ein Reportergen unter der funktionellen Kontrolle von regulatorischen Elementen mindestens eines Globin-Gens enthält, und dass der Nachweis der veränderten Hämoglobinbildung in der Testzelle durch Nachweis des Reportergen-Produktes erfolgt.

10. Verfahren gemäß Anspruch 9, **dadurch gekennzeichnet, dass** das Reportergen Luciferase, alkalische Phosphatase, beta-Galaktosidase oder EGFP ist.

11. Verfahren gemäß Anspruch 9 oder 10, **dadurch gekennzeichnet, dass** das Globin-Gen ein alpha-oder beta-Globin-Gen ist.

12. Verfahren gemäß einem der Ansprüche 1-11, **dadurch gekennzeichnet, dass** die neuronale Testzelle ein primäres Neuron, eine PC 12-Zelle oder eine abgeleitete Neuroblastomzelle ist.

## Claims

1. A screening method for the identification of neuroprotective substances which influence haemoglobin formation in neuronal cells, comprising:
a) bringing the substance to be tested in vitro in contact with a neuronal test cell, and
b) detecting an altered haemoglobin formation in the test cell.

2. The method according to claim 1, wherein the detection of the altered haemoglobin formation is carried out by the detection of at least one globin RNA.

3. The method according to claim 2, wherein the globin RNA to be detected is alpha-globin RNA and/or beta-globin RNA.

4. The method according to claim 2 or 3, wherein the detection of globin RNA is carried out by a polymerase chain reaction (PCR).

5. The method according to claim 2 or 3, wherein the detection of globin RNA is carried out by hybridisation with a suitable probe.

6. The method according to claim 1, wherein the detection of the altered haemoglobin formation is carried out by the detection of haemoglobin protein.

7. The method according to claim 6, wherein the detection of the haemoglobin protein is carried out by staining with 2,7-diaminfluorene or with the cyanohaemoglobin method.

8. The method according to claim 6, wherein the detection of the haemoglobin protein is carried out with an antibody.

9. The method according to claim 1, wherein the test cell contains a reporter gen under functional control of regulatory elements of at least one globin gene, and wherein the detection of the altered haemoglobin formation in the test cell is carried out by the detection of the product of the reporter gene.

10. The method according to claim 9, wherein the reporter gene is luciferase, alkaline phosphatise, beta-galactosidase or EGFP.

11. The method according to claim 9 or 10, wherein the globin gene is a alpha-globin gene or a beta-globin gene.

12. The method according to any of the claims 1 to 11, wherein the neuronal test cell is a primary neuron, a PC12 cell, or a derived neuroblastoma cell.

## Revendications

1. Procédé de criblage pour l'identification de substances protectrices pour cellules neuronales qui influencent la formation d'hémoglobine dans les cellules neuronales, comprenant :
a) la mise en contact *in vitro* de la substance à tester avec une cellule test neuronale, et
b) la détection d'une quantité altérée d'hémo-globine forméedans la cellule test.

2. Procédé selon la revendication 1, **caractérisé en ce que** la détection de la quantité altérée d'hémoglobine formée s'effectue par la détection d'au moins un ARN de globine.

3. Procédé selon la revendication 2, **caractérisé en ce que** l'ARN de globine à détecter est un ARN d'alpha-globine et/ou de bêta-globine.

4. Procédé selon la revendication 2 ou 3, **carac-térisé en ce que** la détection de l'ARN de globine s'effectue au moyen de la réaction en chaîne par polymérase (PCR).

5. Procédé selon la revendication 2 ou 3, **carac-térisé en ce que** la détection de l'ARN de globine se fait par hybridation avec une sonde appropriée.

6. Procédé selon la revendication 1, **caractérisé en ce que** la détection de la quantité altérée d'hémoglobine formée se fait par la détection de la protéine d'hémoglobine.

7. Procédé selon la revendication 6, **caractérisé en ce que** la détection de la protéine d'hémo-globine se fait par coloration avec du 2,7-diaminofluorène ou par la méthode de la cyano-hémoglobine.

8. Procédé selon la revendication 6, **caractérisé en ce que** la détection de la protéine d'hémo-globine s'effectue avec un anticorps.

9. Procédé selon la revendication 1, **caractérisé en ce que** la cellule test contient un gène rap-porteur sous le contrôle fonctionnel des éléments régulateurs d'au moins un gène de la globine, et que la détection de la quantité altérée d'hémo-globine formée dans la cellule test s'effectue par la détection du produit du gène rapporteur.

10. Procédé selon la revendication 9, **caractérisé en ce que** le gène rapporteur est la luciférase, la phosphatase alcaline, la bêta-galactosidase ou l'EGFP.

11. Procédé selon la revendication 9 ou 10, **caractérisé en ce que** le gène de la globine est un gène de l'alpha- ou de la bêta-globine.

12. Procédé selon l'une des revendications 1 à 11, **caractérisé en ce que** la cellule neuronale test est un neurone primaire, une cellule PC12 ou une cellule dérivée de neuroblastome.
